# EUROPÄISCHE PATENTSCHRIFT

(11) **EP 1 527 067 B1**
(45) Veröffentlichungstag und Bekanntmachung des Hinweises auf die Patenterteilung: **04.10.2006**
(21) Anmeldenummer: 03740461.3
(22) Anmeldetag: 14.07.2003
(51) Int. Cl.: C07D 403/14, C07D 401/14, A01N 43/56

(54) **4-TRIFLUORMETHYLPYRAZOLYL SUBSTITUIERTE PYRIDINE UND PYRIMIDINE**
4-TRIFLUOROMETHYLPYRAZOLYL SUBSTITUTED PYRIDINES AND PYRIMIDINES
PYRIDINE ET PYRIMIDINE SUBSTITUEES PAR 4-TRIFLUOROMETHYLPYRAZOLYLE

(30) Priorität: 25.07.2002 DE 10234876
(43) Veröffentlichungstag der Anmeldung: 04.05.2005
(73) Patentinhaber: Bayer CropScience GmbH, 65929 Frankfurt/Main (DE)
(72) Erfinder: HOFFMANN, Michael, Gerhard, 65439 Flörsheim (DE); Dr.HELMKE, Hendrik, 65853 Liederbach (DE); WILLMS, Lothar, 65719 Hofheim (DE); AULER, Thomas, 65812 Bad Soden (DE); BIERINGER, Hermann, 65817 Eppstein (DE); Dr.MENNE, Hubert, 55252 Mainz-Kastel (DE)
(86) Internationale Anmeldenummer: PCT/EP2003/007574
(87) Internationale Veröffentlichungsnummer: WO 2004/013129

(56) Entgegenhaltungen:
- EP-A- 1 101 764
- WO-A-98/40379
- WO-A-99/28301

## Beschreibung

Die Erfindung betrifft das technische Gebiet der Herbizide, insbesondere das der Herbizide aus der Gruppe der Heteroaryl-Pyrazole zur selektiven Bekämpfung von Unkräutern und Ungräsern in Nutzpflanzenkulturen.

Aus verschiedenen Schriften ist bereits bekannt, daß bestimmte durch Azol-Reste, wie Pyrazolyl, Imidazolyl und Triazolyl, substituierte Pyridine und Pyrimidine herbizide Eigenschaften besitzen. So sind aus WO 99/28301 Pyridine und Pyrimidine bekannt, die in 2-Position einen Azol-Rest und in 6-Position einen über ein Kohlenstoffatom gebundenen aromatischen oder heteroaromatischen Rest tragen. WO 98/40379 beschreibt Pyridine und Pyrimidine, die in 2-Position einen Azol-Rest und in 6-Position einen über ein Sauerstoff-, Stickstoff- oder Schwefelatom gebundenen aromatischen oder heteroaromatischen Rest tragen. Der Azol-Rest in 2-Position kann durch verschiedene Reste substituiert sein. Diese Schrift offenbart verschiedene Substituenten für den Pyrazolylrest, die sich stets in 3-Position befinden. EP-A 1 101 764 beschreibt herbizid wirksame 4-Methylpyridine, die in 2-Position durch 3-Trifluormethyl-1-pyrazolyl substituiert sind.

Die aus diesen Schriften bekannten Verbindungen zeigen jedoch häufig eine nicht ausreichende herbizide Wirksamkeit. Aufgabe der vorliegenden Erfindung ist daher die Bereitstellung von herbizid wirksamen Verbindungen mit - gegenüber den im Stand der Technik offenbarten Verbindungen - verbesserten herbiziden Eigenschaften.

Es wurde nun gefunden, daß bestimmte durch 4-Trifluormethylpyrazolyl substituierte Pyridine und Pyrimidine als Herbizide besonders gut geeignet sind. Ein Gegenstand der vorliegenden Erfindung sind daher Verbindungen der Formel (I), deren N-Oxide und deren Salze, 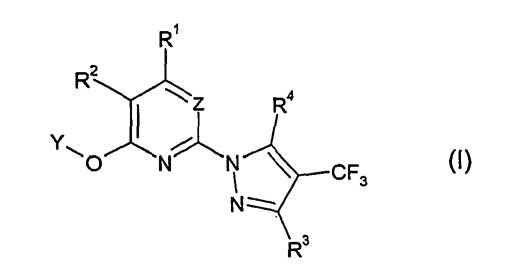 worin die Reste und Indizes folgende Bedeutungen haben:
- Z: bedeutet N oder CR⁸;
- Y: bedeutet einen Rest aus der Gruppe Y1 bisY7: 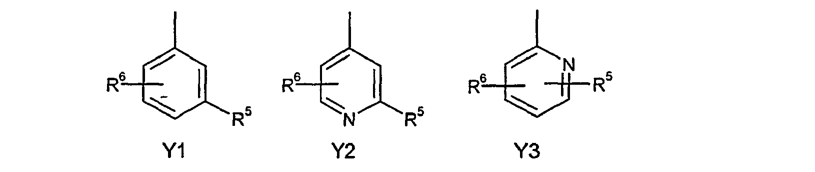 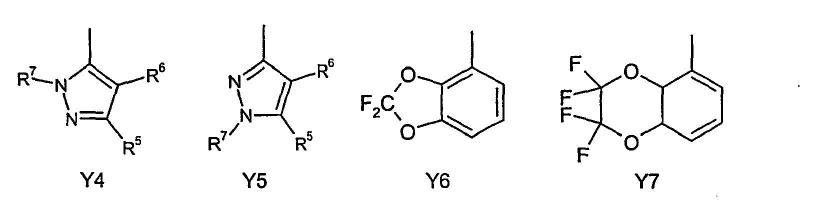
- R¹ und R²: bedeuten unabhängig voneinander Wasserstoff, Halogen, Cyano, Isocyano, OH, COOR¹⁰, COR¹⁰, CH₂OH, CH₂SH, CH₂NH₂, NO₂, CSNH₂, CONH₂, (C₁-C₄)-Alkyl, Halogen-(C₁-C₄)-alkyl, (C₃-C₆)-Cycloalkyl, (C₁-C₄)-Alkoxy, Halogen-(C₁-C₄)-alkoxy, (C₁-C₂)-Alkoxy-(C₁-C₂)-alkyl, (C₂-C₄)-Alkenyl, (C₂-C₄)-Alkinyl, (C₃-C₄)-Alkenyloxy, (C₃-C₄)-Alkinyloxy, (C₁-C₂)-Alkylthio-(C₁-C₂)-alkyl, S(O)ₙR⁹, (C₁-C₂)-Alkylsulfonyl-(C₁-C₂)-alkyl, Amino, (C₁-C₄)-Alkylamino, (C₁-C₃)-Alkylcarbonylamino, (C₁-C₄)-Alkylsulfonylamino oder Di-(C₁-C₄)-Alkylamino;
- R³ und R⁴: bedeuten unabhängig voneinander Wasserstoff, Halogen, Cyano, (C₁₋C₄)-Alkyl, Halogen-(C₁-C₄)-alkyl, (C₁-C₄)-Alkoxy oder Halogen-(C₁-C₄)-alkoxy;
- R⁵: bedeutet Halogen, Cyano, (C₁-C₄)-Alkyl, Halogen-(C₁-C₄)-alkyl, (C₁-C₄)-Alkoxy, Halogen-(C₁-C₄)-alkoxy, Halogen-(C₁-C₄)-alkylthio, (C₃-C₅)-Cycloalkyl, Halogen-(C₃-C₅)-Cycloalkyl, SF₅, S(O)ₙR⁹, (C₂-C₄)-Alkenyl oder (C₂-C₄)-Alkinyl;
- R⁶: bedeutet Wasserstoff, Halogen, Cyano, (C₁-C₄)-Alkyl, Halogen-(C₁-C₄)-alkyl, (C₁-C₄)-Alkoxy, Halogen-(C₁-C₄)-alkoxy oder S(O)ₙR⁹;
- R⁷: bedeutet (C₁-C₄)-Alkyl;
- R⁸: bedeutet Wasserstoff, Halogen, Cyano, NO₂, (C₁-C₄)-Alkyl, (C₁-C₄)-Alkoxy, Hydroxy, Amino, (C₁-C₄)-Alkylamino, (C₁-C₃)-Alkylcarbonylamino, (C₁-C₄)-Alkylsulfonylamino, Di-(C₁-C₄)-Alkylamino oder S(O)ₙR⁹;
- R⁹: bedeutet Wasserstoff, (C₁-C₄)-Alkyl oder Halogen-(C₁-C₄)-alkyl;
- R¹⁰: bedeutet Wasserstoff oder (C₁-C₄)-Alkyl;
- n: bedeutet 0, 1 oder 2.

In Formel (I) und allen nachfolgenden Formeln können Alkyl-, Alkenyl- und Alkinylreste mit mehr als zwei beziehungsweise drei C-Atomen geradkettig oder verzweigt sein. Alkylreste bedeuten Methyl, Ethyl, n- oder i-Propyl, n-, i-, t- oder 2-Butyl. Alkenyl bedeutet demgemäß Ethenyl, 1-Propenyl, 2-Propenyl sowie die verschiedenen Butenyl-Isomeren. Alkinyl bedeutet Ethinyl, 1-Propinyl, 2-Propinyl sowie die verschiedenen Butinyl-Isomeren. Analog sind die Defintionen in ihren zusammengesetzten Bedeutungen wie Alkoxy, Alkenyloxy, Alkinyloxy und Alkylthio zu verstehen. So steht Alkinyloxy beispielsweise für HC≡CCH₂O, CH₃C≡CCH₂O und CH₃C≡CCH₂CH₂O.

Cycloalkyl bedeutet Cyclopropyl, Cyclobutyl, Cyclopentyl oder Cyclohexyl.

Im Falle einer zweifach substituierten Aminogruppe, wie Dialkylamino, können diese beiden Substituenten gleich oder verschieden sein.

Halogen bedeutet Fluor, Chlor, Brom oder Iod. Halogenalkyl bedeutet durch Halogen, vorzugsweise durch Fluor, Chlor und/oder Brom, insbesondere durch Fluor oder Chlor, teilweise oder vollständig substituiertes Alkyl, z.B. CF₃, CHF₂, CH₂F, CF₃CF₂, CH₂FCHCl, CCl₃, CHCl₂, CH₂CH₂Cl; Halogenalkoxy ist z.B. OCF₃, OCHF₂, OCH₂F, CF₃CF₂O, OCH₂CF₃ und OCH₂CH₂Cl; entsprechendes gilt für andere durch Halogen substituierte Reste.

Die Verbindungen der Formel (I) können je nach Art und Verknüpfung der Substituenten als Stereoisomere vorliegen. Liegt beispielsweise eine Doppelbindung vor, können Diastereomere auftreten. Sind beispielsweise ein oder mehrere asymmetrische C-Atome vorhanden, so können Enantiomere und Diastereomere auftreten. Stereoisomere lassen sich aus den bei der Herstellung anfallenden Gemischen nach üblichen Trennmethoden, z.B. durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Verbindungen der Formel (I) können grundsätzlich N-Oxide bilden. N-Oxide können gemäß dem Fachmann bekannten Methoden durch Umsetzung mit oxidierenden Reagenzien wie Persäuren, Wasserstoffperoxid und Natriumperborat hergestellt werden. Solche Methoden sind beispielsweise in T.L. Gilchrist, Comprehensive Organic Synthesis, Volume 7, Seiten 748 bis 750, S.V. Ley, Ed., Pergamon Press beschrieben.

Verbindungen der Formel (I) können grundsätzlich Salze bilden durch Addition mit
a) Säuren wie Chlorwasserstoff, Bromwasserstoff, Salpetersäure, Phosphorsäure, Schwefelsäure, Essigsäure, Oxalsäure, oder
b) Basen wie Pyridin, Ammoniak, Triethylamin, Natriumcarbonat, Kaliumcarbonat, Natriumhydroxid, Kaliumhydroxid.

Bevorzugte Ausführungsformen der erfindungsgemäßen Verbindungen umfassen, soweit im Folgenden nicht anders vermerkt, stets die N-Oxide und Salze.

Von besonderem Interesse sind Verbindungen der Formel (I), worin Y einen Rest aus der Gruppe Y1 bis Y6 bedeutet.

Als vorteilhaft haben sich Verbindungen der Formel (I) herausgestellt, worin R¹ und R² unabhängig voneinander Wasserstoff, Halogen, Cyano, OH, CHO, Vinyl, (C₁-C₄)-Alkyl, Halogen-(C₁-C₄)-Alkyl, Vinyl oder (C₁-C₄)-Alkoxy bedeuten, und die anderen Substituenten und Indices jeweils die weiter oben genannten Bedeutungen haben.

Ebenfalls von Vorteil sind Verbindungen der allgemeinen Formel (I), worin R³ und R⁴ unabhängig voneinander Wasserstoff, Halogen, Methyl oder Methoxy bedeuten, und die anderen Substituenten und Indices jeweils die weiter oben genannten Bedeutungen haben.

Bevorzugt sind Verbindungen der allgemeinen Formel (I), worin
- R¹: Wasserstoff, Halogen, Cyano, CHO, Methoxy, Methyl oder Ethyl und
- R²: Wasserstoff, OH, Methyl, Ethyl, Methoxy oder Ethoxy bedeuten, und die anderen Substituenten und Indices jeweils die weiter oben genannten Bedeutungen haben.

Bevorzugt sind auch Verbindungen der allgemeinen Formel (I), worin R³ und R⁴ jeweils Wasserstoff oder Methyl bedeuten, und die anderen Substituenten und Indices jeweils die weiter oben genannten Bedeutungen haben.

Besonders bevorzugt sind Verbindungen der allgemeinen Formel (I), worin R⁸ für Wasserstoff, Halogen oder (C₁-C₄)-Alkyl steht, und die anderen Substituenten und Indices jeweils die weiter oben genannten Bedeutungen haben.

Ebenfalls besonders bevorzugt sind Verbindungen der allgemeinen Formel (I), worin R⁵ Halogen, Cyano, Halogen-(C₁-C₄)-alkyl, Halogen-(C₁-C₄)-alkoxy oder Halogen-(C₁-C₄)-alkylthio bedeutet, und die anderen Substituenten und Indices jeweils die weiter oben genannten Bedeutungen haben.

Weiterhin besonders bevorzugt sind Verbindungen der allgemeinen Formel (I), worin R⁶ Wasserstoff bedeutet, und die anderen Substituenten und Indices jeweils die weiter oben genannten Bedeutungen haben.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise nach den in den folgenden Schemata angegebenen Reaktionswegen hergestellt werden:

Nach Schema 1 können Verbindungen der Formel (IIa), in der E¹ für eine Fluchtgruppe wie Halogen, Methylsulfonyl oder Tosyl steht, unter Basenkatalyse mit einer Verbindung der Formel (III) umgesetzt werden. Solche Reaktionen sind dem Fachmann bekannt.

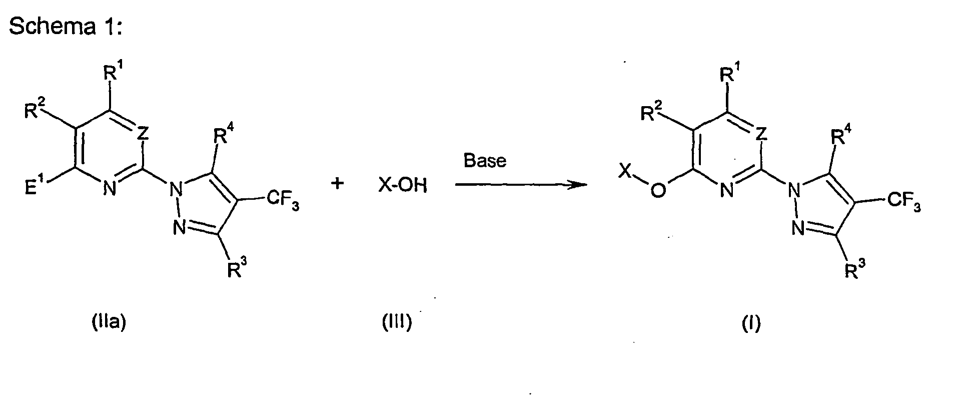

Verbindungen der Formel (IIa), in der E¹ für Halogen steht, können beispielsweise nach Schema 2 unter Basenkatalyse aus einer Verbindung der Formel (IV) mit einem Pyrazol der Formel (V) hergestellt werden. Dabei können die Regioisomere (IIa) und (IIb) entstehen, die beispielsweise durch chromatographische Aufarbeitung getrennt werden können. Solche Reaktionen sind dem Fachmann bekannt.

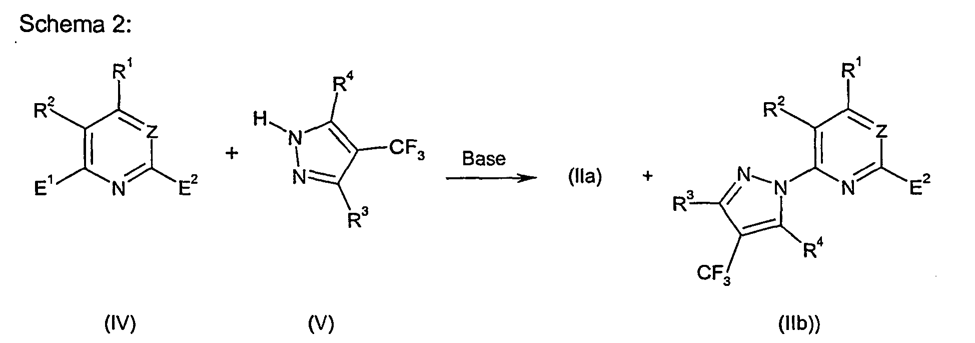

Verbindungen der Formel (IIa), in der E¹ für Methylsulfonyl steht, können beispielsweise nach Schema 3 durch Oxidation mit m-Chlorperbenzoesäure (MCPA) oder Oxone® aus einer Verbindung der Formel (IIc) hergestellt werden. Solche Reaktionen sind dem Fachmann beispielsweise aus J. March, Advanced Organic Chemistry, John Wiley, New York, 1992, 4^{th} Ed., Seiten 1201 bis 1203 bekannt.

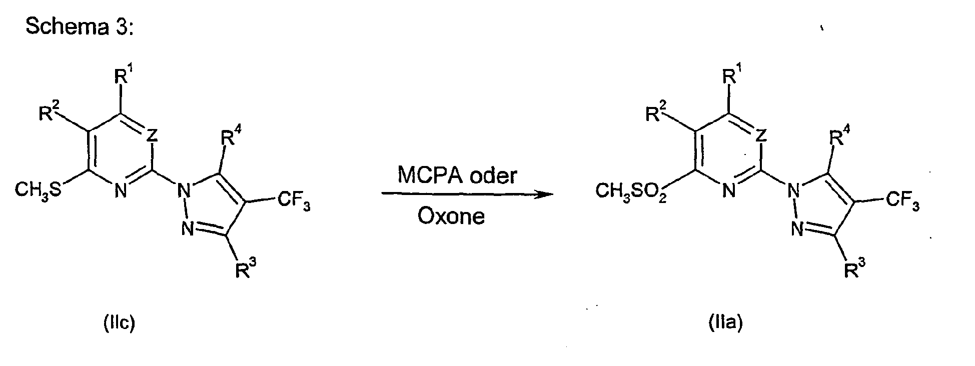

Verbindungen der Formel (IIb) können beispielsweise nach Schema 4 durch basenkatalysierte Umsetzung einer Verbindung der Formel (VI) mit dem Pyrazol (V) hergestellt werden. Als Basen eignen sich die Carbonate von Kalium und Natrium, die Hydroxide von Kalium und Natrium sowie Natriumhydrid. Zweckmäßigerweise werden diese Reaktionen in Lösungsmitteln wie Dimethylformamid, Dioxan, THF, Sulfolan und Acetonitril durchgeführt. Solche Reaktionen sind dem Fachmann bekannt.

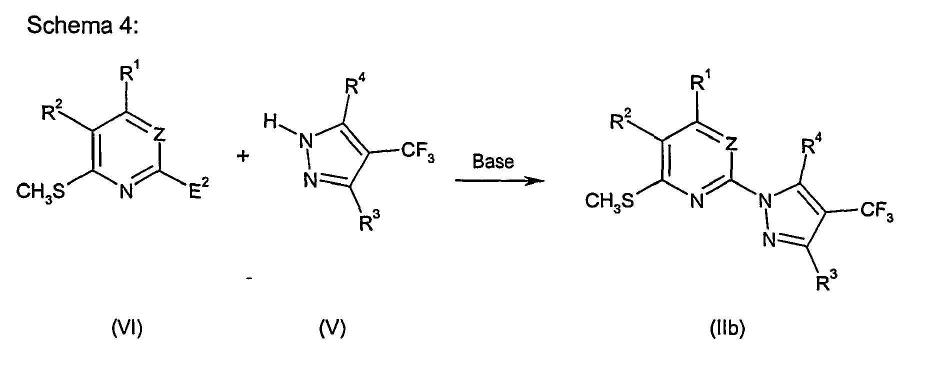

Verbindungen der Formel (VI) können beispielsweise aus Verbindungen der Formel (IV), in der E¹ und E² jeweils für Halogen stehen, durch Umsetzung mit einem Natrium- oder Kaliumsalz von Methylmercaptan in Tetrahydrofuran oder Dioxan hergestellt werden. Solche Reaktionen sind dem Fachmann bekannt.

Verbindungen der Formel (IV), in der E¹ und E² jeweils für Halogen stehen, sind entweder kommerziell erhältlich oder können gemäß dem Fachmann bekannten Methoden hergestellt werden. Solche dem Fachmann bekannten Methoden werden beispielsweise beschrieben in Advances in Heterocyclic Chemistry, Katritzky, A.R., Ed., Academic Press, New York, 1993, Volume 58, Seiten 301 bis 305; Heterocyclic Compounds, Elderfield, R.C., Ed., John Wiley, New York, 1957, Volume 6, Seiten 265 bis 270.

Pyrazole der Formel (V) können gemäß dem Fachmann bekannten Mehoden hergestellt werden. Die Darstellung von 4-Trifluormethylpyrazol ist beispielsweise in THL, 37, 11, 1996 Seite 1829-1832 beschrieben.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, lpomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Amaranthus retroflexus, Avena sp., Echinochloa sp., Cyperus serotinus, Lolium multiflorum, Setaria viridis, Sagittaria pygmaea, Scirpus juncoides, Sinapis sp. und Stellaria media.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Weizen, Gerste, Mais, Reis und Sojabohne auf. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Emtegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (SP), wasserlösliche Pulver (WP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können z.B. verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäure-ester oder Polyoxethylensorbitanester wie z.B. Polyoxyethylen-sorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICl-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und - methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung von 5-Methyl-4-(3-trifluormethylphenoxy)-2-(4-trifluormethyl-1H-1-pyrazolyl)pyrimidin:

Eine Mischung aus 11.2 g (36.4 mmol) 5-Methyl-4-methylsulfonyl-2-(4-trifluormethyl-1H-1-pyrazolyl)pyrimidin, 7.7g (47.4 mmol) 3-Trifluormethylphenol und 10.1g (72.9 mmol) K₂CO₃ in 200 ml DMF wird 24 h bei RT gerührt. Danach wird auf 200 ml Wasser gegossen und viermal mit jeweils 100 ml CH₂Cl₂ extrahiert. Die vereinigte organische Phase wird über Na₂SO₄ getrocknet, abfiltriert und eingeengt. Chromatographische Reinigung an Kieselgel mit Laufmittel Heptan/Essigester (1:1) ergibt 10.2g (72%) 5-Methyl-4-(3-trifluormethylphenoxy)-2-(4-trifluormethyl-1H-1-pyrazolyl)pyrimidin als farblose Kristalle mit einem Festpunkt von 103-105°C.
¹H-NMR: δ [CDCl₃] 2.40 (s, 3H), 7.45 (m, 1H), 7.55 (s, 1H), 7.62 (m, 2H), 7.92 (s, 1H), 8.33 (s, 1H), 8.52 (s, 1H).

### Herstellung von 5-Methyl-2-(4-trifluormethyl)-1H-1-pyrazolyl-4-(2-trifluormethyl-4-pyridyloxy)pyrimidin:

Eine Mischung aus 0.38g (1.23 mmol) 5-Methyl-4-methylsulfonyl-2-(4-trifluormethyl-1H-1-pyrazolyl)pyrimidin, 0.2g (1.23 mmol) 2-Trifluormethyl-4-hydroxypyridin und 0.33g (2.45 mmol) K₂CO₃ in 10 ml DMF wird 6 h bei 60°C und dann 48 h bei RT gerührt. Danach wird auf 20 ml Wasser gegossen und viermal mit jeweils 15 ml CH₂Cl₂ extrahiert. Die vereinigte organische Phase wird über Na₂SO₄ getrocknet, abfiltriert und eingeengt. Chromatographische Reinigung an Kieselgel mit Laufmittel Heptan/Essigester (3:7) ergibt 0.16g (33%) 5-Methyl-2-(4-trifluormethyl)-1H-1-pyrazolyl-4-(2-trifluormethyl-4-pyridyloxy)pyrimidin als hellgelbes Öl.
¹H-NMR: δ [CDCl₃] 2.40 (s, 3H), 7.50 (dd, 1H), 7.70 (d, 1H), 7.95 (s, 1H), 8.50 (s, 1H), 8.60 (s, 1H), 8.85 (d, 1H).

### Herstellung von 2-(1-Methyl-3-trifluormethylpyrazol-5-yl-oxy)-6-(4-trifluormethylpyrazol-1-yl)pyridin:

0.262g 4-Trifluormethylpyrazol werden unter Stickstoff in 7 ml Dimethylacetamid vorgelegt und bei 0°C mit 0.057g NaH versetzt. Man läßt innerhalb von 30 min auf RT kommen und setzt dann 0.5 g 2-Fluor-6-(1-methyl-3-trifluormethylpyrazol-5-yloxy)pyridin zu und erwärmt für 7 h auf 140°C, kühlt auf RT ab und gießt auf Wasser. Nach zweimaliger Extraktion mit Essigester/Heptan (1:1) wird mit Wasser und gesättigter Kochsalzlösung gewaschen, über MgSO₄ getrocknet und eingeengt. Chromatographische Reinigung an Kieselgel ergibt 0.349 g 2-(1-Methyl-3-trifluormethylpyrazol-5-yl-oxy)-6-(4-trifluormethylpyrazol-1-yl)pyridin als weisse Kristalle.
¹H-NMR: δ [CDCl₃] 3.82 (s, 3H), 6.34 (s, 1H), 7.00 (d, 1H), 7.82 (d, 1H), 7.88 (s, 1H), 7.97 (t, 1H), 8.43 (s, 1H).

### Herstellung von 4-Methyl-2-(1-methyl-3-trifluormethylpyrazol-5-yl-oxy)-6-(4-trifluormethylpyrazol-1-yl)pyridin:

0.385g 4-Trifluormethylpyrazol werden unter Stickstoff in 10 ml Dimethylacetamid vorgelegt und bei 0°C mit 0.096g NaH versetzt. Man läßt innerhalb von 30 min auf RT kommen und setzt dann 0.757 g 2-Chlor-4-methyl-6-(1-methyl-3-trifluormethylpyrazol-5-yl-oxy)pyridin zu und erwärmt für 7 h auf 140°C, kühlt auf RT ab und gießt auf Wasser. Nach jeweils zweimaliger Extraktion mit Essigester/Heptan (1:1) und Essigester wird mit Wasser und gesättigter Kochsalzlösung gewaschen, über MgSO₄ getrocknet und eingeengt.
Chromatographische Reinigung an Kieselgel ergibt 0.332g 4-Methyl-2-(1-methyl-3-trifluormethylpyrazol-5-yl-oxy)-6-(4-trifluormethylpyrazol-1-yl)pyridin als weisse Kristalle.
¹H-NMR: δ [CDCl₃] 2.50 (s, 3H), 3.82 (s, 3H), 6.30 (s, 1H), 6.82 (d, 1H), 7.67 (s, 1H), 7.86 (s, 1H), 8.43 (s, 1H).

### Herstellung von 4-Methoxy-2-(1-methyl-3-trifluormethylpyrazol-5-yl-oxy)-6-(4-trifluormethylpyrazoi-1-yl)pyridin:

0.068g 4-Trifluormethylpyrazol werden unter Stickstoff in 5 ml Dimethylacetamid vorgelegt und bei 0°C mit 0.017g NaH versetzt. Man läßt innerhalb von 30 min auf RT kommen und setzt dann 0.2g 4-Methoxy-2,6-bis-(1-methyl-3-trifluormethylpyrazol-5-yl-oxy)pyridin zu und erwärmt für 5 h auf 135°C, kühlt auf RT ab und gießt auf Wasser. Nach dreimaliger Extraktion mit Essigester/Heptan (1:1) wird mit Wasser und gesättigter Kochsalzlösung gewaschen, über MgSO₄ getrocknet und eingeengt.
Chromatographische Reinigung an Kieselgel ergibt 0.036g 4-Methoxy-2-(1-methyl-3-trifluormethylpyrazol-5-yl-oxy)-6-(4-trifluormethylpyrazol-1-yl)pyridin als wachsartige Substanz.
¹H-NMR: δ [CDCl₃] 3.81 (s, 3H), 3.99 (s, 3H), 6.29 (s, 1H), 6.44 (d, 1H), 7.40 (d, 1H), 7.85 (s, 1H), 8.42 (s, 1H).

Die in nachfolgenden Tabellen aufgeführten Beispiele wurden analog oben genannten Methoden hergestellt beziehungsweise sind analog den oben genannten Methoden erhältlich.

Die hier verwendeten Abkürzungen bedeuten:

| | | |
|---|---|---|
| Et = Ethyl | OEt = Ethoxy | Me = Methyl |
| OMe = Methoxy | EE = Essigester | Fp. = Festpunkt |
| R^{f} = Retentionswert | i-Pr = iso-Propyl | n-Pr = n-Propyl |
| RT = Raumtemperatur | | |

**Tabelle 1: Erfindungsgemäße Verbindungen der allgemeinen Formel (I), worin die Substituenten und Symbole folgende Bedeutungen haben:**

| | | | | | | | |
|---|---|---|---|---|---|---|---|
| 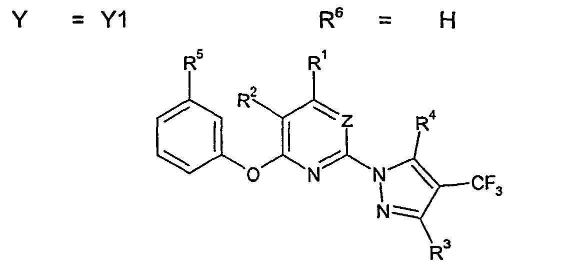 | | | | | | | |

| **Nr.** | **R**^{**1**} | **R**^{**2**} | **R**^{**3**} | **R**^{**4**} | **R**^{**5**} | **Z** | **Physikal. Daten** |
|---|---|---|---|---|---|---|---|
| 1.1 | H | H | H | H | CF₃ | N | ¹H-NMR (CDCl₃): 6.90 (d, 1H); 7.45 (m, 1H); 7.55 (m, 1H); 7.62 (m, 2H); 7,98 (s, 1H); 8.50 (s, 1H); 8.70 (d, 1H) |
| 1.2 | H | H | H | H | CF₃ | CH | Fp. 55-56°C |
| 1.3 | H | H | H | H | CF₃ | CMe | |
| 1.4 | H | H | H | H | CF₃ | CCl | |
| 1.5 | H | H | Me | H | CF₃ | CH | |
| 1.6 | H | OH | H | Me | CF₃ | CH | |
| 1.7 | H | Me | H | H | CF₃ | N | Fp.103-105°C |
| 1.8 | H | OMe | H | H | CF₃ | CH | |
| 1.9 | H | H | H | H | Cl | N | |
| 1.10 | H | H | H | H | Cl | CH | |
| 1.11 | H | H | H | H | Cl | CMe | |
| 1.12 | H | H | H | H | Cl | CCl | |
| 1.13 | H | H | Me | H | Cl | CH | |
| 1.14 | H | OH | H | Me | Cl | CH | |
| 1.15 | H | Me | H | H | Cl | CH | |
| 1.16 | H | OMe | H | H | Cl | CH | |
| 1.17 | H | H | H | H | CN | N | |
| 1.18 | H | H | H | H | CN | CH | Fp.86-88°C |
| 1.19 | H | H | H | H | CN | CMe | |
| 1.20 | H | H | H | H | CN | CCl | |
| 1.21 | H | H | Me | H | CN | CH | |
| 1.22 | H | OH | H | Me | CN | CH | |
| 1.23 | H | Me | H | H | CN | CH | |
| 1.24 | H | OMe | H | H | CN | CH | |
| 1.25 | H | H | H | H | OCF₂H | N | |
| 1.26 | H | H | H | H | OCF₂H | CH | |
| 1.27 | H | H | H | H | OCF₂H | CMe | |
| 1.28 | H | H | H | H | OCF₂H | CCl | |
| 1.29 | H | H | Me | H | OCF₂H | CH | |
| 1.30 | H | OH | H | Me | OCF₂H | CH | |
| 1.31 | H | Me | H | H | OCF₂H | CH | |
| 1.32 | H | OMe | H | H | OCF₂H | CH | |
| 1.33 | H | H | H | H | CN | N | |
| 1.34 | H | H | H | H | CN | CH | |
| 1.35 | H | H | H | H | CN | CMe | |
| 1.36 | H | H | H | H | CN | CCl | |
| 1.37 | H | H | Me | H | CN | CH | |
| 1.38 | H | OH | H | Me | CN | CH | |
| 1.39 | H | Me | H | H | CN | CH | |
| 1.40 | H | OMe | H | H | CN | CH | |
| 1.41 | H | H | H | H | CN | N | |
| 1.42 | H | H | H | H | CN | CH | |
| 1.43 | H | H | H | H | CN | CMe | |
| 1.44 | H | H | H | H | CN | CCl | |
| 1.45 | H | H | Me | H | CN | CH | |
| 1.46 | H | OH | H | Me | CN | CH | |
| 1.47 | H | Me | H | H | CN | CH | |
| 1.48 | H | OMe | H | H | CN | CH | |
| 1.49 | Me | H | H | H | CF₃ | N | Fp. 109-111°C |
| 1.50 | Me | H | H | H | CF₃ | CH | |
| 1.51 | Me | H | H | H | CF₃ | CMe | |
| 1.52 | Me | H | H | H | CF₃ | CCl | |
| 1.53 | Me | H | Me | H | CF₃ | CH | |
| 1.54 | Me | OH | H | Me | CF₃ | CH | |
| 1.55 | Me | Me | H | H | CF₃ | CH | |
| 1.56 | Me | OMe | H | H | CF₃ | CH | |
| 1.57 | Me | H | H | H | Cl | N | |
| 1.58 | Me | H | H | H | Cl | CH | |
| 1.59 | Me | H | H | H | Cl | CMe | |
| 1.60 | Me | H | H | H | Cl | CCl | |
| 1.61 | Me | H | Me | H | Cl | CH | |
| 1.62 | Me | OH | H | Me | Cl | CH | |
| 1.63 | Me | Me | H | H | Cl | CH | |
| 1.64 | Me | OMe | H | H | Cl | CH | |
| 1.65 | Me | H | H | H | CN | N | |
| 1.66 | Me | H | H | H | CN | CH | |
| 1.67 | Me | H | H | H | CN | CMe | |
| 1.68 | Me | H | H | H | CN | CCl | |
| 1.69 | Me | H | Me | H | CN | CH | |
| 1.70 | Me | OH | H | Me | CN | CH | |
| 1.71 | Me | Me | H | H | CN | CH | |
| 1.72 | Me | OMe | H | H | CN | CH | |
| 1.73 | Me | H | H | H | OCF₂H | N | |
| 1.74 | Me | H | H | H | OCF₂H | CH | |
| 1.75 | Me | H | H | H | OCF₂H | CMe | |
| 1.76 | Me | H | H | H | OCF₂H | CCl | |
| 1.77 | Me | H | Me | H | OCF₂H | CH | |
| 1.78 | Me | OH | H | Me | OCF₂H | CH | |
| 1.79 | Me | Me | H | H | OCF₂H | CH | |
| 1.80 | Me | OMe | H | H | OCF₂H | CH | |
| 1.81 | Me | H | H | H | CN | N | |
| 1.82 | Me | H | H | H | CN | CH | |
| 1.83 | Me | H | H | H | CN | CMe | |
| 1.84 | Me | H | H | H | CN | CCl | |
| 1.85 | Me | H | Me | H | CN | CH | |
| 1.86 | Me | OH | H | Me | CN | CH | |
| 1.87 | Me | Me | H | H | CN | CH | |
| 1.88 | Me | OMe | H | H | CN | CH | |
| 1.89 | Me | H | H | H | CN | N | |
| 1.90 | Me | H | H | H | CN | CH | |
| 1.91 | Me | H | H | H | CN | CMe | |
| 1.92 | Me | H | H | H | CN | CCl | |
| 1.93 | Me | H | Me | H | CN | CH | |
| 1.94 | Me | OH | H | Me | CN | CH | |
| 1.95 | Me | Me | H | H | CN | CH | |
| 1.96 | Me | OMe | H | H | CN | CH | |
| 1.97 | CN | H | H | H | CF₃ | N | |
| 1.98 | CN | H | H | H | CF₃ | CH | |
| 1.99 | CN | H | H | H | CF₃ | CMe | |
| 1.100 | CN | H | H | H | CF₃ | CCl | |
| 1.101 | CN | H | Me | H | CF₃ | CH | |
| 1.102 | CN | OH | H | Me | CF₃ | CH | |
| 1.103 | CN | Me | H | H | CF₃ | CH | |
| 1.104 | CN | OMe | H | H | CF₃ | CH | |
| 1.105 | CN | H | H | H | Cl | N | |
| 1.106 | CN | H | H | H | Cl | CH | |
| 1.107 | CN | H | H | H | Cl | CMe | |
| 1.108 | CN | H | H | H | Cl | CCl | |
| 1.109 | CN | H | Me | H | Cl | CH | |
| 1.110 | CN | OH | H | Me | Cl | CH | |
| 1.111 | CN | Me | H | H | Cl | CH | |
| 1.112 | CN | OMe | H | H | Cl | CH | |
| 1.113 | CN | H | H | H | CN | N | |
| 1.114 | CN | H | H | H | CN | CH | |
| 1.115 | CN | H | H | H | CN | CMe | |
| 1.116 | CN | H | H | H | CN | CCl | |
| 1.117 | CN | H | Me | H | CN | CH | |
| 1.118 | CN | OH | H | Me | CN | CH | |
| 1.119 | CN | Me | H | H | CN | CH | |
| 1.120 | CN | OMe | H | H | CN | CH | |
| 1.121 | CN | H | H | H | OCF₂H | N | |
| 1.122 | CN | H | H | H | OCF₂H | CH | |
| 1.123 | CN | H | H | H | OCF₂H | CMe | |
| 1.124 | CN | H | H | H | OCF₂H | CCl | |
| 1.125 | CN | H | Me | H | OCF₂H | CH | |
| 1.126 | CN | OH | H | Me | OCF₂H | CH | |
| 1.127 | CN | Me | H | H | OCF₂H | CH | |
| 1.128 | CN | OMe | H | H | OCF₂H | CH | |
| 1.129 | CN | H | H | H | CN | N | |
| 1.130 | CN | H | H | H | CN | CH | |
| 1.131 | CN | H | H | H | CN | CMe | |
| 1.132 | CN | H | H | H | CN | CCl | |
| 1.133 | CN | H | Me | H | CN | CH | |
| 1.134 | CN | OH | H | Me | CN | CH | |
| 1.135 | CN | Me | H | H | CN | CH | |
| 1.136 | CN | OMe | H | H | CN | CH | |
| 1.137 | CN | H | H | H | CN | N | |
| 1.138 | CN | H | H | H | CN | CH | |
| 1.139 | CN | H | H | H | CN | CMe | |
| 1.140 | CN | H | H | H | CN | CCl | |
| 1.141 | CN | H | Me | H | CN | CH | |
| 1.142 | CN | OH | H | Me | CN | CH | |
| 1.143 | CN | Me | H | H | CN | CH | |
| 1.144 | CN | OMe | H | H | CN | CH | |
| 1.145 | OMe | H | H | H | CF₃ | N | |
| 1.146 | OMe | H | H | H | CF₃ | CH | |
| 1.147 | OMe | H | H | H | CF₃ | CMe | |
| 1.148 | OMe | H | H | H | CF₃ | CCl | |
| 1.149 | OMe | H | Me | H | CF₃ | CH | |
| 1.150 | OMe | OH | H | Me | CF₃ | CH | |
| 1.151 | OMe | Me | H | H | CF₃ | CH | |
| 1.152 | OMe | OMe | H | H | CF₃ | CH | |
| 1.153 | OMe | H | H | H | Cl | N | |
| 1.154 | OMe | H | H | H | Cl | CH | |
| 1.155 | OMe | H | H | H | Cl | CMe | |
| 1.156 | OMe | H | H | H | Cl | CCl | |
| 1.157 | OMe | H | Me | H | Cl | CH | |
| 1.158 | OMe | OH | H | Me | Cl | CH | |
| 1.159 | OMe | Me | H | H | Cl | CH | |
| 1.160 | OMe | OMe | H | H | Cl | CH | |
| 1.161 | OMe | H | H | H | CN | N | |
| 1.162 | OMe | H | H | H | CN | CH | |
| 1.163 | OMe | H | H | H | CN | CMe | |
| 1.164 | OMe | H | H | H | CN | CCl | |
| 1.165 | OMe | H | Me | H | CN | CH | |
| 1.166 | OMe | OH | H | Me | CN | CH | |
| 1.167 | OMe | Me | H | H | CN | CH | |
| 1.168 | OMe | OMe | H | H | CN | CH | |
| 1.169 | OMe | H | H | H | OCF₂H | N | |
| 1.170 | OMe | H | H | H | OCF₂H | CH | |
| 1.171 | OMe | H | H | H | OCF₂H | CMe | |
| 1.172 | OMe | H | H | H | OCF₂H | CCl | |
| 1.173 | OMe | H | Me | H | OCF₂H | CH | |
| 1.174 | OMe | OH | H | Me | OCF₂H | CH | |
| 1.175 | OMe | Me | H | H | OCF₂H | CH | |
| 1.176 | OMe | OMe | H | H | OCF₂H | CH | |
| 1.177 | OMe | H | H | H | CN | N | |
| 1.178 | OMe | H | H | H | CN | CH | |
| 1.179 | OMe | H | H | H | CN | CMe | |
| 1.180 | OMe | H | H | H | CN | CCl | |
| 1.181 | OMe | H | Me | H | CN | CH | |
| 1.182 | OMe | OH | H | Me | CN | CH | |
| 1.183 | OMe | Me | H | H | CN | CH | |
| 1.184 | OMe | OMe | H | H | CN | CH | |
| 1.185 | OMe | H | H | H | CN | N | |
| 1.186 | OMe | H | H | H | CN | CH | |
| 1.187 | OMe | H | H | H | CN | CMe | |
| 1.188 | OMe | H | H | H | CN | CCl | |
| 1.189 | OMe | H | Me | H | CN | CH | |
| 1.190 | OMe | OH | H | Me | CN | CH | |
| 1.191 | OMe | Me | H | H | CN | CH | |
| 1.192 | OMe | OMe | H | H | CN | CH | |
| 1.193 | H | OMe | H | H | CF₃ | N | Fp. 100-102°C |
| 1.194 | H | Cl | H | H | CF₃ | N | Fp. 91-93°C |
| 1.195 | H | Me | Me | H | CF₃ | N | Fp. 97-99°C |

**Tabelle 2: Erfindungsgemäße Verbindungen der allgemeinen Formel (I), worin die Substituenten und Symbole folgende Bedeutungen haben:**

| | | | | | | | |
|---|---|---|---|---|---|---|---|
| 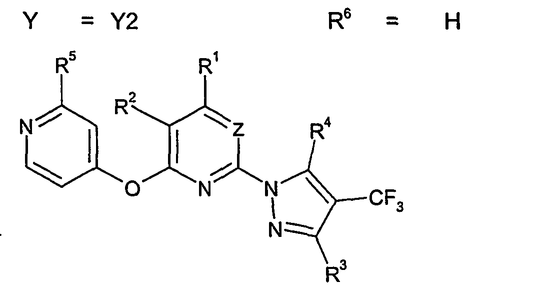 | | | | | | | |

| **Nr.** | **R**^{**1**} | **R**^{**2**} | **R**^{**3**} | **R**^{**4**} | **R**^{**5**} | **Z** | **Physikal. Daten** |
|---|---|---|---|---|---|---|---|
| 2.1 | H | H | H | H | CF₃ | N | |
| 2.2 | H | H | H | H | CF₃ | CH | Fp.73-74°C |
| 2.3 | H | H | H | H | CF₃ | CMe | |
| 2.4 | H | H | H | H | CF₃ | CCl | |
| 2.5 | H | H | Me | H | CF₃ | CH | Fp.73-75°C |
| 2.6 | H | OH | H | Me | CF₃ | CH | |
| 2.7 | H | Me | H | H | CF₃ | N | ¹H-NMR: δ [CDCl₃] 2.40 (s, 3H), 7.50 (dd, 1H), 7.70 (d, 1H), 7.95 (s, 1H), 8.50 (s, 1H), 8.60 (s, 1H), 8.85 (d, 1H). |
| 2.8 | H | OMe | H | H | CF₃ | CH | |
| 2.9 | H | H | H | H | Cl | N | |
| 2.10 | H | H | H | H | Cl | CH | |
| 2.11 | H | H | H | H | Cl | CMe | |
| 2.12 | H | H | H | H | Cl | CCl | |
| 2.13 | H | H | Me | H | Cl | CH | |
| 2.14 | H | OH | H | Me | Cl | CH | |
| 2.15 | H | Me | H | H | Cl | CH | |
| 2.16 | H | OMe | H | H | Cl | CH | |
| 2.17 | H | H | H | H | CN | N | |
| 2.18 | H | H | H | H | CN | CH | |
| 2.19 | H | H | H | H | CN | CMe | |
| 2.20 | H | H | H | H | CN | CCl | |
| 2.21 | H | H | Me | H | CN | CH | |
| 2.22 | H | OH | H | Me | CN | CH | |
| 2.23 | H | Me | H | H | CN | CH | |
| 2.24 | H | OMe | H | H | CN | CH | |
| 2.25 | H | H | H | H | OCF₂H | N | |
| 2.26 | H | H | H | H | OCF₂H | CH | |
| 2.27 | H | H | H | H | OCF₂H | CMe | |
| 2.28 | H | H | H | H | OCF₂H | CCl | |
| 2.29 | H | H | Me | H | OCF₂H | CH | |
| 2.30 | H | OH | H | Me | OCF₂H | CH | |
| 2.31 | H | Me | H | H | OCF₂H | CH | |
| 2.32 | H | OMe | H | H | OCF₂H | CH | |
| 2.33 | H | H | H | H | CN | N | |
| 2.34 | H | H | H | H | CN | CH | |
| 2.35 | H | H | H | H | CN | CMe | |
| 2.36 | H | H | H | H | CN | CCl | |
| 2.37 | H | H | Me | H | CN | CH | |
| 2.38 | H | OH | H | Me | CN | CH | |
| 2.39 | H | Me | H | H | CN | CH | |
| 2.40 | H | OMe | H | H | CN | CH | |
| 2.41 | H | H | H | H | CN | N | |
| 2.42 | H | H | H | H | CN | CH | |
| 2.43 | H | H | H | H | CN | CMe | |
| 2.44 | H | H | H | H | CN | CCl | |
| 2.45 | H | H | Me | H | CN | CH | |
| 2.46 | H | OH | H | Me | CN | CH | |
| 2.47 | H | Me | H | H | CN | CH | |
| 2.48 | H | OMe | H | H | CN | CH | |
| 2.49 | Me | H | H | H | CF₃ | N | |
| 2.50 | Me | H | H | H | CF₃ | CH | |
| 2.51 | Me | H | H | H | CF₃ | CMe | |
| 2.52 | Me | H | H | H | CF₃ | CCl | |
| 2.53 | Me | H | Me | H | CF₃ | CH | |
| 2.54 | Me | OH | H | Me | CF₃ | CH | |
| 2.55 | Me | Me | H | H | CF₃ | CH | |
| 2.56 | Me | OMe | H | H | CF₃ | CH | |
| 2.57 | Me | H | H | H | Cl | N | |
| 2.58 | Me | H | H | H | Cl | CH | |
| 2.59 | Me | H | H | H | Cl | CMe | |
| 2.60 | Me | H | H | H | Cl | CCl | |
| 2.61 | Me | H | Me | H | Cl | CH | |
| 2.62 | Me | OH | H | Me | Cl | CH | |
| 2.63 | Me | Me | H | H | Cl | CH | |
| 2.64 | Me | OMe | H | H | Cl | CH | |
| 2.65 | Me | H | H | H | CN | N | |
| 2.66 | Me | H | H | H | CN | CH | |
| 2.67 | Me | H | H | H | CN | CMe | |
| 2.68 | Me | H | H | H | CN | CCl | |
| 2.69 | Me | H | Me | H | CN | CH | |
| 2.70 | Me | OH | H | Me | CN | CH | |
| 2.71 | Me | Me | H | H | CN | CH | |
| 2.72 | Me | OMe | H | H | CN | CH | |
| 2.73 | Me | H | H | H | OCF₂H | N | |
| 2.74 | Me | H | H | H | OCF₂H | CH | |
| 2.75 | Me | H | H | H | OCF₂H | CMe | |
| 2.76 | Me | H | H | H | OCF₂H | CCl | |
| 2.77 | Me | H | Me | H | OCF₂H | CH | |
| 2.78 | Me | OH | H | Me | OCF₂H | CH | |
| 2.79 | Me | Me | H | H | OCF₂H | CH | |
| 2.80 | Me | OMe | H | H | OCF₂H | CH | |
| 2.81 | Me | H | H | H | CN | N | |
| 2.82 | Me | H | H | H | CN | CH | |
| 2.83 | Me | H | H | H | CN | CMe | |
| 2.84 | Me | H | H | H | CN | CCl | |
| 2.85 | Me | H | Me | H | CN | CH | |
| 2.86 | Me | OH | H | Me | CN | CH | |
| 2.87 | Me | Me | H | H | CN | CH | |
| 2.88 | Me | OMe | H | H | CN | CH | |
| 2.89 | Me | H | H | H | CN | N | |
| 2.90 | Me | H | H | H | CN | CH | |
| 2.91 | Me | H | H | H | CN | CMe | |
| 2.92 | Me | H | H | H | CN | CCl | |
| 2.93 | Me | H | Me | H | CN | CH | |
| 2.94 | Me | OH | H | Me | CN | CH | |
| 2.95 | Me | Me | H | H | CN | CH | |
| 2.96 | Me | OMe | H | H | CN | CH | |
| 2.97 | CN | H | H | H | CF₃ | N | Fp. 115-116°C |
| 2.98 | CN | H | H | H | CF₃ | CH | |
| 2.99 | CN | H | H | H | CF₃ | CMe | |
| 2.100 | CN | H | H | H | CF₃ | CCl | |
| 2.101 | CN | H | Me | H | CF₃ | CH | |
| 2.102 | CN | OH | H | Me | CF₃ | CH | |
| 2.103 | CN | Me | H | H | CF₃ | CH | |
| 2.104 | CN | OMe | H | H | CF₃ | CH | |
| 2.105 | CN | H | H | H | Cl | N | |
| 2.106 | CN | H | H | H | Cl | CH | |
| 2.107 | CN | H | H | H | Cl | CMe | |
| 2.108 | CN | H | H | H | Cl | CCl | |
| 2.109 | CN | H | Me | H | Cl | CH | |
| 2.110 | CN | OH | H | Me | Cl | CH | |
| 2.111 | CN | Me | H | H | Cl | CH | |
| 2.112 | CN | OMe | H | H | Cl | CH | |
| 2.113 | CN | H | H | H | CN | N | |
| 2.114 | CN | H | H | H | CN | CH | |
| 2.115 | CN | H | H | H | CN | CMe | |
| 2.116 | CN | H | H | H | CN | CCl | |
| 2.117 | CN | H | Me | H | CN | CH | |
| 2.118 | CN | OH | H | Me | CN | CH | |
| 2.119 | CN | Me | H | H | CN | CH | |
| 2.120 | CN | OMe | H | H | CN | CH | |
| 2.121 | CN | H | H | H | OCF₂H | N | |
| 2.122 | CN | H | H | H | OCF₂H | CH | |
| 2.123 | CN | H | H | H | OCF₂H | CMe | |
| 2.124 | CN | H | H | H | OCF₂H | CCl | |
| 2.125 | CN | H | Me | H | OCF₂H | CH | |
| 2.126 | CN | OH | H | Me | OCF₂H | CH | |
| 2.127 | CN | Me | H | H | OCF₂H | CH | |
| 2.128 | CN | OMe | H | H | OCF₂H | CH | |
| 2.129 | CN | H | H | H | CN | N | |
| 2.130 | CN | H | H | H | CN | CH | |
| 2.131 | CN | H | H | H | CN | CMe | |
| 2.132 | CN | H | H | H | CN | CCl | |
| 2.133 | CN | H | Me | H | CN | CH | |
| 2.134 | CN | OH | H | Me | CN | CH | |
| 2.135 | CN | Me | H | H | CN | CH | |
| 2.136 | CN | OMe | H | H | CN | CH | |
| 2.137 | CN | H | H | H | CN | N | |
| 2.138 | CN | H | H | H | CN | CH | |
| 2.139 | CN | H | H | H | CN | CMe | |
| 2.140 | CN | H | H | H | CN | CCl | |
| 2.141 | CN | H | Me | H | CN | CH | |
| 2.142 | CN | OH | H | Me | CN | CH | |
| 2.143 | CN | Me | H | H | CN | CH | |
| 2.144 | CN | OMe | H | H | CN | CH | |
| 2.145 | OMe | H | H | H | CF₃ | N | |
| 2.146 | OMe | H | H | H | CF₃ | CH | |
| 2.147 | OMe | H | H | H | CF₃ | CMe | |
| 2.148 | OMe | H | H | H | CF₃ | CCl | |
| 2.149 | OMₑ | H | Me | H | CF₃ | CH | |
| 2.150 | OMe | OH | H | Me | CF₃ | CH | |
| 2.151 | OMe | Me | H | H | CF₃ | CH | |
| 2.152 | OMe | OMe | H | H | CF₃ | CH | |
| 2.153 | OMe | H | H | H | Cl | N | |
| 2.154 | OMe | H | H | H | Cl | CH | |
| 2.155 | OMe | H | H | H | Cl | CMe | |
| 2.156 | OMe | H | H | H | Cl | CCl | |
| 2.157 | OMe | H | Me | H | Cl | CH | |
| 2.158 | OMe | OH | H | Me | Cl | CH | |
| 2.159 | OMe | Me | H | H | Cl | CH | |
| 2.160 | OMe | OMe | H | H | Cl | CH | |
| 2.161 | OMe | H | H | H | CN | N | |
| 2.162 | OMe | H | H | H | CN | CH | |
| 2.163 | OMe | H | H | H | CN | CMe | |
| 2.164 | OMe | H | H | H | CN | CCl | |
| 2.165 | OMe | H | Me | H | CN | CH | |
| 2.166 | OMe | OH | H | Me | CN | CH | |
| 2.167 | OMe | Me | H | H | CN | CH | |
| 2.168 | OMe | OMe | H | H | CN | CH | |
| 2.169 | OMe | H | H | H | OCF₂H | N | |
| 2.170 | OMe | H | H | H | OCF₂H | CH | |
| 2.171 | OMe | H | H | H | OCF₂H | CMe | |
| 2.172 | OMe | H | H | H | OCF₂H | CCl | |
| 2.173 | OMe | H | Me | H | OCF₂H | CH | |
| 2.174 | OMe | OH | H | Me | OCF₂H | CH | |
| 2.175 | OMe | Me | H | H | OCF₂H | CH | |
| 2.176 | OMe | OMe | H | H | OCF₂H | CH | |
| 2.177 | OMe | H | H | H | CN | N | |
| 2.178 | OMe | H | H | H | CN | CH | |
| 2.179 | OMe | H | H | H | CN | CMe | |
| 2.180 | OMe | H | H | H | CN | CCl | |
| 2.181 | OMe | H | Me | H | CN | CH | |
| 2.182 | OMe | OH | H | Me | CN | CH | |
| 2.183 | OMe | Me | H | H | CN | CH | |
| 2.184 | OMe | OMe | H | H | CN | CH | |
| 2.185 | OMe | H | H | H | CN | N | |
| 2.186 | OMe | H | H | H | CN | CH | |
| 2.187 | OMe | H | H | H | CN | CMe | |
| 2.188 | OMe | H | H | H | CN | CCl | |
| 2.189 | OMe | H | Me | H | CN | CH | |
| 2.190 | OMe | OH | H | Me | CN | CH | |
| 2.191 | OMe | Me | H | H | CN | CH | |
| 2.192 | OMe | OMe | H | H | CN | CH | |
| 2.193 | H | OMe | H | H | CF₃ | N | Fp. 137-139°C |
| 2.194 | H | Br | H | H | CF₃ | N | Fp. 101-103°C |
| 2.195 | H | Cl | H | H | CF₃ | N | Fp.119-121°C |
| 2.196 | H | Me | H | Me | CF₃ | N | ¹H-NMR: 2.10 (s, 3H); 2.40 (s, 3H); 6.95 (dd, 1H); 7.20 (d, 1H); 7,75 (s, 1H); 8.35 (d, 1H); 8.60 (s, 1H) |

**Tabelle 3: Erfindungsgemäße Verbindungen der allgemeinen Formel (I), worin die Substituenten und Symbole folgende Bedeutungen haben:**

| | | | | | | | |
|---|---|---|---|---|---|---|---|
| 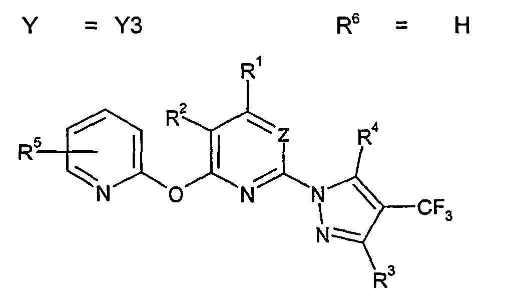 | | | | | | | |

| **Nr.** | **R**^{**1**} | **R**^{**2**} | **R**^{**3**} | **R**^{**4**} | **R**^{**5**} | **Z** | **Physikal. Daten** |
|---|---|---|---|---|---|---|---|
| 3.1 | H | H | H | H | CF₃ | N | |
| 3.2 | H | H | H | H | CF₃ | CH | |
| 3.3 | H | H | H | H | CF₃ | CMe | |
| 3.4 | H | H | H | H | CF₃ | CCl | |
| 3.5 | H | H | Me | H | CF₃ | CH | |
| 3.6 | H | OH | H | Me | CF₃ | CH | |
| 3.7 | H | Me | H | H | CF₃ | CH | |
| 3.8 | H | OMe | H | H | CF₃ | CH | |
| 3.9 | H | H | H | H | Cl | N | |
| 3.10 | H | H | H | H | Cl | CH | |
| 3.11 | H | H | H | H | Cl | CMe | |
| 3.12 | H | H | H | H | Cl | CCl | |
| 3.13 | H | H | Me | H | Cl | CH | |
| 3.14 | H | OH | H | Me | Cl | CH | |
| 3.15 | H | Me | H | H | Cl | CH | |
| 3.16 | H | OMe | H | H | Cl | CH | |
| 3.17 | H | H | H | H | CN | N | |
| 3.18 | H | H | H | H | CN | CH | |
| 3.19 | H | H | H | H | CN | CMe | |
| 3.20 | H | H | H | H | CN | CCl | |
| 3.21 | H | H | Me | H | CN | CH | |
| 3.22 | H | OH | H | Me | CN | CH | |
| 3.23 | H | Me | H | H | CN | CH | |
| 3.24 | H | OMe | H | H | CN | CH | |
| 3.25 | H | H | H | H | OCF₂H | N | |
| 3.26 | H | H | H | H | OCF₂H | CH | |
| 3.27 | H | H | H | H | OCF₂H | CMe | |
| 3.28 | H | H | H | H | OCF₂H | CCl | |
| 3.29 | H | H | Me | H | OCF₂H | CH | |
| 3.30 | H | OH | H | Me | OCF₂H | CH | |
| 3.31 | H | Me | H | H | OCF₂H | CH | |
| 3.32 | H | OMe | H | H | OCF₂H | CH | |
| 3.33 | H | H | H | H | CN | N | |
| 3.34 | H | H | H | H | CN | CH | |
| 3.35 | H | H | H | H | CN | CMe | |
| 3.36 | H | H | H | H | CN | CCl | |
| 3.37 | H | H | Me | H | CN | CH | |
| 3.38 | H | OH | H | Me | CN | CH | |
| 3.39 | H | Me | H | H | CN | CH | |
| 3.40 | H | OMe | H | H | CN | CH | |
| 3.41 | H | H | H | H | CN | N | |
| 3.42 | H | H | H | H | CN | CH | |
| 3.43 | H | H | H | H | CN | CMe | |
| 3.44 | H | H | H | H | CN | CCl | |
| 3.45 | H | H | Me | H | CN | CH | |
| 3.46 | H | OH | H | Me | CN | CH | |
| 3.47 | H | Me | H | H | CN | CH | |
| 3.48 | H | OMe | H | H | CN | CH | |
| 3.49 | Me | H | H | H | CF₃ | N | |
| 3.50 | Me | H | H | H | CF₃ | CH | |
| 3.51 | Me | H | H | H | CF₃ | CMe | |
| 3.52 | Me | H | H | H | CF₃ | CCl | |
| 3.53 | Me | H | Me | H | CF₃ | CH | |
| 3.54 | Me | OH | H | Me | CF₃ | CH | |
| 3.55 | Me | Me | H | H | CF₃ | CH | |
| 3.56 | Me | OMe | H | H | CF₃ | CH | |
| 3.57 | Me | H | H | H | Cl | N | |
| 3.58 | Me | H | H | H | Cl | CH | |
| 3.59 | Me | H | H | H | Cl | CMe | |
| 3.60 | Me | H | H | H | Cl | CCl | |
| 3.61 | Me | H | Me | H | Cl | CH | |
| 3.62 | Me | OH | H | Me | Cl | CH | |
| 3.63 | Me | Me | H | H | Cl | CH | |
| 3.64 | Me | OMe | H | H | Cl | CH | |
| 3.65 | Me | H | H | H | CN | N | |
| 3.66 | Me | H | H | H | CN | CH | |
| 3.67 | Me | H | H | H | CN | CMe | |
| 3.68 | Me | H | H | H | CN | CCl | |
| 3.69 | Me | H | Me | H | CN | CH | |
| 3.70 | Me | OH | H | Me | CN | CH | |
| 3.71 | Me | Me | H | H | CN | CH | |
| 3.72 | Me | OMe | H | H | CN | CH | |
| 3.73 | Me | H | H | H | OCF₂H | N | |
| 3.74 | Me | H | H | H | OCF₂H | CH | |
| 3.75 | Me | H | H | H | OCF₂H | CMe | |
| 3.76 | Me | H | H | H | OCF₂H | CCl | |
| 3.77 | Me | H | Me | H | OCF₂H | CH | |
| 3.78 | Me | OH | H | Me | OCF₂H | CH | |
| 3.79 | Me | Me | H | H | OCF₂H | CH | |
| 3.80 | Me | OMe | H | H | OCF₂H | CH | |
| 3.81 | Me | H | H | H | CN | N | |
| 3.82 | Me | H | H | H | CN | CH | |
| 3.83 | Me | H | H | H | CN | CMe | |
| 3.84 | Me | H | H | H | CN | CCl | |
| 3.85 | Me | H | Me | H | CN | CH | |
| 3.86 | Me | OH | H | Me | CN | CH | |
| 3.87 | Me | Me | H | H | CN | CH | |
| 3.88 | Me | OMe | H | H | CN | CH | |
| 3.89 | Me | H | H | H | CN | N | |
| 3.90 | Me | H | H | H | CN | CH | |
| 3.91 | Me | H | H | H | CN | CMe | |
| 3.92 | Me | H | H | H | CN | CCl | |
| 3.93 | Me | H | Me | H | CN | CH | |
| 3.94 | Me | OH | H | Me | CN | CH | |
| 3.95 | Me | Me | H | H | CN | CH | |
| 3.96 | Me | OMe | H | H | CN | CH | |
| 3.97 | CN | H | H | H | CF₃ | N | |
| 3.98 | CN | H | H | H | CF₃ | CH | |
| 3.99 | CN | H | H | H | CF₃ | CMe | |
| 3.100 | CN | H | H | H | CF₃ | CCl | |
| 3.101 | CN | H | Me | H | CF₃ | CH | |
| 3.102 | CN | OH | H | Me | CF₃ | CH | |
| 3.103 | CN | Me | H | H | CF₃ | CH | |
| 3.104 | CN | OMe | H | H | CF₃ | CH | |
| 3.105 | CN | H | H | H | Cl | N | |
| 3.106 | CN | H | H | H | Cl | CH | |
| 3.107 | CN | H | H | H | Cl | CMe | |
| 3.108 | CN | H | H | H | Cl | CCl | |
| 3.109 | CN | H | Me | H | Cl | CH | |
| 3.110 | CN | OH | H | Me | Cl | CH | |
| 3.111 | CN | Me | H | H | Cl | CH | |
| 3.112 | CN | OMe | H | H | Cl | CH | |
| 3.113 | CN | H | H | H | CN | N | |
| 3.114 | CN | H | H | H | CN | CH | |
| 3.115 | CN | H | H | H | CN | CMe | |
| 3.116 | CN | H | H | H | CN | CCl | |
| 3.117 | CN | H | Me | H | CN | CH | |
| 3.118 | CN | OH | H | Me | CN | CH | |
| 3.119 | CN | Me | H | H | CN | CH | |
| 3.120 | CN | OMe | H | H | CN | CH | |
| 3.121 | CN | H | H | H | OCF₂H | N | |
| 3.122 | CN | H | H | H | OCF₂H | CH | |
| 3.123 | CN | H | H | H | OCF₂H | CMe | |
| 3.124 | CN | H | H | H | OCF₂H | CCl | |
| 3.125 | CN | H | Me | H | OCF₂H | CH | |
| 3.126 | CN | OH | H | Me | OCF₂H | CH | |
| 3.127 | CN | Me | H | H | OCF₂H | CH | |
| 3.128 | CN | OMe | H | H | OCF₂H | CH | |
| 3.129 | CN | H | H | H | CN | N | |
| 3.130 | CN | H | H | H | CN | CH | |
| 3.131 | CN | H | H | H | CN | CMe | |
| 3.132 | CN | H | H | H | CN | CCl | |
| 3.133 | CN | H | Me | H | CN | CH | |
| 3.134 | CN | OH | H | Me | CN | CH | |
| 3.135 | CN | Me | H | H | CN | CH | |
| 3.136 | CN | OMe | H | H | CN | CH | |
| 3.137 | CN | H | H | H | CN | N | |
| 3.138 | CN | H | H | H | CN | CH | |
| 3.139 | CN | H | H | H | CN | CMe | |
| 3.140 | CN | H | H | H | CN | CCl | |
| 3.141 | CN | H | Me | H | CN | CH | |
| 3.142 | CN | OH | H | Me | CN | CH | |
| 3.143 | CN | Me | H | H | CN | CH | |
| 3.144 | CN | OMe | H | H | CN | CH | |
| 3.145 | OMe | H | H | H | CF₃ | N | |
| 3.146 | OMe | H | H | H | CF₃ | CH | |
| 3.147 | OMe | H | H | H | CF₃ | CMe | |
| 3.148 | OMe | H | H | H | CF₃ | CCl | |
| 3.149 | OMe | H | Me | H | CF₃ | CH | |
| 3.150 | OMe | OH | H | Me | CF₃ | CH | |
| 3.151 | OMe | Me | H | H | CF₃ | CH | |
| 3.152 | OMe | OMe | H | H | CF₃ | CH | |
| 3.153 | OMe | H | H | H | Cl | N | |
| 3.154 | OMe | H | H | H | Cl | CH | |
| 3.155 | OMe | H | H | H | Cl | CMe | |
| 3.156 | OMe | H | H | H | Cl | CCl | |
| 3.157 | OMe | H | Me | H | Cl | CH | |
| 3.158 | OMe | OH | H | Me | Cl | CH | |
| 3.159 | OMe | Me | H | H | Cl | CH | |
| 3.160 | OMe | OMe | H | H | Cl | CH | |
| 3.161 | OMe | H | H | H | CN | N | |
| 3.162 | OMe | H | H | H | CN | CH | |
| 3.163 | OMe | H | H | H | CN | CMe | |
| 3.164 | OMe | H | H | H | CN | CCl | |
| 3.165 | OMe | H | Me | H | CN | CH | |
| 3.166 | OMe | OH | H | Me | CN | CH | |
| 3.167 | OMe | Me | H | H | CN | CH | |
| 3.168 | OMe | OMe | H | H | CN | CH | |
| 3.169 | OMe | H | H | H | OCF₂H | N | |
| 3.170 | OMe | H | H | H | OCF₂H | CH | |
| 3.171 | OMe | H | H | H | OCF₂H | CMe | |
| 3.172 | OMe | H | H | H | OCF₂H | CCl | |
| 3.173 | OMe | H | Me | H | OCF₂H | CH | |
| 3.174 | OMe | OH | H | Me | OCF₂H | CH | |
| 3.175 | OMe | Me | H | H | OCF₂H | CH | |
| 3.176 | OMe | OMe | H | H | OCF₂H | CH | |
| 3.177 | OMe | H | H | H | CN | N | |
| 3.178 | OMe | H | H | H | CN | CH | |
| 3.179 | OMe | H | H | H | CN | CMe | |
| 3.180 | OMe | H | H | H | CN | CCl | |
| 3.181 | OMe | H | Me | H | CN | CH | |
| 3.182 | OMe | OH | H | Me | CN | CH | |
| 3.183 | OMe | Me | H | H | CN | CH | |
| 3.184 | OMe | OMe | H | H | CN | CH | |
| 3.185 | OMe | H | H | H | CN | N | |
| 3.186 | OMe | H | H | H | CN | CH | |
| 3.187 | OMe | H | H | H | CN | CMe | |
| 3.188 | OMe | H | H | H | CN | CCl | |
| 3.189 | OMe | H | Me | H | CN | CH | |
| 3.190 | OMe | OH | H | Me | CN | CH | |
| 3.191 | OMe | Me | H | H | CN | CH | |
| 3.192 | OMe | OMe | H | H | CN | CH | |

**Tabelle 4: Erfindungsgemäße Verbindungen der allgemeinen Formel (I), worin die Substituenten und Symbole folgende Bedeutungen haben:**

| | | | | | | | |
|---|---|---|---|---|---|---|---|
| 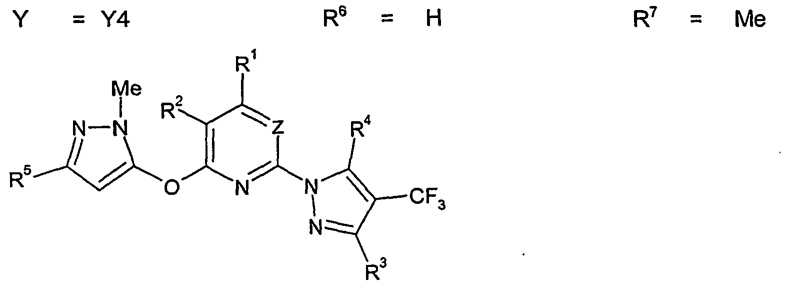 | | | | | | | |

| **Nr.** | **R**^{**1**} | **R**^{**2**} | **R**^{**3**} | **R**^{**4**} | **R**^{**5**} | **Z** | **Physikal. Daten** |
|---|---|---|---|---|---|---|---|
| 4.1 | H | H | H | H | CF₃ | N | ¹H-NMR: δ [CDCl₃] 3.82 (s, 3H), 6.60 (s, 1H), 7.05 (d, 1H), 8.00 (s, 1H), 8.65 (s, 1H), 8.80 (d, 1H) |
| 4.2 | H | H | H | H | CF₃ | CH | ¹H NMR (CDCl₃/TMS): δ (ppm) = 8.43 (s, 1H), 7.97 (t, 1H), 7.88 (s, 1H), 7.82 (d, 1H), 7.00 (d, 1H), 6.34 (s, 1H), 3.82 (s, 3H). |
| 4.3 | H | H | H | H | CF₃ | CMe | |
| 4.4 | H | H | H | H | CF₃ | CCl | |
| 4.5 | H | H | Me | H | CF₃ | CH | Fp. 95-96°C |
| 4.6 | H | OH | H | Me | CF₃ | CH | |
| 4.7 | H | Me | H | H | CF₃ | CH | |
| 4.8 | H | OMe | H | H | CF₃ | CH | |
| 4.9 | H | H | H | H | Cl | N | |
| 4.10 | H | H | H | H | Cl | CH | |
| 4.11 | H | H | H | H | Cl | CMe | |
| 4.12 | H | H | H | H | Cl | CCl | |
| 4.13 | H | H | Me | H | Cl | CH | |
| 4.14 | H | OH | H | Me | Cl | CH | |
| 4.15 | H | Me | H | H | Cl | CH | |
| 4.16 | H | OMe | H | H | Cl | CH | |
| 4.17 | H | H | H | H | CN | N | |
| 4.18 | H | H | H | H | CN | CH | |
| 4.19 | H | H | H | H | CN | CMe | |
| 4.20 | H | H | H | H | CN | CCl | |
| 4.21 | H | H | Me | H | CN | CH | |
| 4.22 | H | OH | H | Me | CN | CH | |
| 4.23 | H | Me | H | H | CN | CH | |
| 4.24 | H | OMe | H | H | CN | CH | |
| 4.25 | H | H | H | H | OCF₂H | N | |
| 4.26 | H | H | H | H | OCF₂H | CH | |
| 4.27 | H | H | H | H | OCF₂H | CMe | |
| 4.28 | H | H | H | H | OCF₂H | CCl | |
| 4.29 | H | H | Me | H | OCF₂H | CH | |
| 4.30 | H | OH | H | Me | OCF₂H | CH | |
| 4.31 | H | Me | H | H | OCF₂H | CH | |
| 4.32 | H | OMe | H | H | OCF₂H | CH | |
| 4.33 | H | H | H | H | CN | N | |
| 4.34 | H | H | H | H | CN | CH | |
| 4.35 | H | H | H | H | CN | CMe | |
| 4.36 | H | H | H | H | CN | CCl | |
| 4.37 | H | H | Me | H | CN | CH | |
| 4.38 | H | OH | H | Me | CN | CH | |
| 4.39 | H | Me | H | H | CN | CH | |
| 4.40 | H | OMe | H | H | CN | CH | |
| 4.41 | H | H | H | H | CN | N | |
| 4.42 | H | H | H | H | CN | CH | |
| 4.43 | H | H | H | H | CN | CMe | |
| 4.44 | H | H | H | H | CN | CCl | |
| 4.45 | H | H | Me | H | CN | CH | |
| 4.46 | H | OH | H | Me | CN | CH | |
| 4.47 | H | Me | H | H | CN | CH | |
| 4.48 | H | OMe | H | H | CN | CH | |
| 4.49 | Me | H | H | H | CF₃ | N | Fp.122-124°C |
| 4.50 | Me | H | H | H | CF₃ | CH | ¹H NMR (CDCl₃/TMS): δ (ppm) 8.43 (s, 1H), 7.86 (s, 1H), 7.67 (s, 1H), 6.82 (s, 1H,), 6.30 (s, 1H), 3.82 (s, 3H), 2.50 (s, 3H). |
| 4.51 | Me | H | H | H | CF₃ | CMe | |
| 4.52 | Me | H | H | H | CF₃ | CCl | |
| 4.53 | Me | H | Me | H | CF₃ | CH | |
| 4.54 | Me | OH | H | Me | CF₃ | CH | |
| 4.55 | Me | Me | H | H | CF₃ | CH | |
| 4.56 | Me | OMe | H | H | CF₃ | CH | |
| 4.57 | Me | H | H | H | Cl | N | |
| 4.58 | Me | H | H | H | Cl | CH | |
| 4.59 | Me | H | H | H | Cl | CMe | |
| 4.60 | Me | H | H | H | Cl | CCl | |
| 4.61 | Me | H | Me | H | Cl | CH | |
| 4.62 | Me | OH | H | Me | Cl | CH | |
| 4.63 | Me | Me | H | H | Cl | CH | |
| 4.64 | Me | OMe | H | H | Cl | CH | |
| 4.65 | Me | H | H | H | CN | N | |
| 4.66 | Me | H | H | H | CN | CH | |
| 4.67 | Me | H | H | H | CN | CMe | |
| 4.68 | Me | H | H | H | CN | CCl | |
| 4.69 | Me | H | Me | H | CN | CH | |
| 4.70 | Me | OH | H | Me | CN | CH | |
| 4.71 | Me | Me | H | H | CN | CH | |
| 4.72 | Me | OMe | H | H | CN | CH | |
| 4.73 | Me | H | H | H | OCF₂H | N | |
| 4.74 | Me | H | H | H | OCF₂H | CH | |
| 4.75 | Me | H | H | H | OCF₂H | CMe | |
| 4.76 | Me | H | H | H | OCF₂H | CCl | |
| 4.77 | Me | H | Me | H | OCF₂H | CH | |
| 4.78 | Me | OH | H | Me | OCF₂H | CH | |
| 4.79 | Me | Me | H | H | OCF₂H | CH | |
| 4.80 | Me | OMe | H | H | OCF₂H | CH | |
| 4.81 | Me | H | H | H | CN | N | |
| 4.82 | Me | H | H | H | CN | CH | |
| 4.83 | Me | H | H | H | CN | CMe | |
| 4.84 | Me | H | H | H | CN | CCl | |
| 4.85 | Me | H | Me | H | CN | CH | |
| 4.86 | Me | OH | H | Me | CN | CH | |
| 4.87 | Me | Me | H | H | CN | CH | |
| 4.88 | Me | OMe | H | H | CN | CH | |
| 4.89 | Me | H | H | H | CN | N | |
| 4.90 | Me | H | H | H | CN | CH | |
| 4.91 | Me | H | H | H | CN | CMe | |
| 4.92 | Me | H | H | H | CN | CCl | |
| 4.93 | Me | H | Me | H | CN | CH | |
| 4.94 | Me | OH | H | Me | CN | CH | |
| 4.95 | Me | Me | H | H | CN | CH | |
| 4.96 | Me | OMe | H | H | CN | CH | |
| 4.97 | CN | H | H | H | CF₃ | N | |
| 4.98 | CN | H | H | H | CF₃ | CH | R_{f} = 0.63; Heptan/Essigester 1:1 |
| 4.99 | CN | H | H | H | CF₃ | CMe | |
| 4.100 | CN | H | H | H | CF₃ | CCl | |
| 4.101 | CN | H | Me | H | CF₃ | CH | ¹H-NMR [CDCl₃]: 2.42 (s, 3H); 3.86 (s, 3H); 6.36 (s, 1H); 7.19 (s, 1H); 8.02 (s, 1H); 8.30(s, 1H) |
| 4.102 | CN | OH | H | Me | CF₃ | CH | |
| 4.103 | CN | Me | H | H | CF₃ | CH | |
| 4.104 | CN | OMe | H | H | CF₃ | CH | |
| 4.105 | CN | H | H | H | Cl | N | |
| 4.106 | CN | H | H | H | Cl | CH | |
| 4.107 | CN | H | H | H | Cl | CMe | |
| 4.108 | CN | H | H | H | Cl | CCl | |
| 4.109 | CN | H | Me | H | Cl | CH | |
| 4.110 | CN | OH | H | Me | Cl | CH | |
| 4.111 | CN | Me | H | H | Cl | CH | |
| 4.112 | CN | OMe | H | H | Cl | CH | |
| 4.113 | CN | H | H | H | CN | N | |
| 4.114 | CN | H | H | H | CN | CH | |
| 4.115 | CN | H | H | H | CN | CMe | |
| 4.116 | CN | H | H | H | CN | CCI | |
| 4.117 | CN | H | Me | H | CN | CH | |
| 4.118 | CN | OH | H | Me | CN | CH | |
| 4.119 | CN | Me | H | H | CN | CH | |
| 4.120 | CN | OMe | H | H | CN | CH | |
| 4.121 | CN | H | H | H | OCF₂H | N | |
| 4.122 | CN | H | H | H | OCF₂H | CH | |
| 4.123 | CN | H | H | H | OCF₂H | CMe | |
| 4.124 | CN | H | H | H | OCF₂H | CCI | |
| 4.125 | CN | H | Me | H | OCF₂H | CH | |
| 4.126 | CN | OH | H | Me | OCF₂H | CH | |
| 4.127 | CN | Me | H | H | OCF₂H | CH | |
| 4.128 | CN | OMe | H | H | OCF₂H | CH | |
| 4.129 | CN | H | H | H | CN | N | |
| 4.130 | CN | H | H | H | CN | CH | |
| 4.131 | CN | H | H | H | CN | CMe | |
| 4.132 | CN | H | H | H | CN | CCl | |
| 4.133 | CN | H | Me | H | CN | CH | |
| 4.134 | CN | OH | H | Me | CN | CH | |
| 4.135 | CN | Me | H | H | CN | CH | |
| 4.136 | CN | OMe | H | H | CN | CH | |
| 4.137 | CN | H | H | H | CN | N | |
| 4.138 | CN | H | H | H | CN | CH | |
| 4.139 | CN | H | H | H | CN | CMe | |
| 4.140 | CN | H | H | H | CN | CCl | |
| 4.141 | CN | H | Me | H | CN | CH | |
| 4.142 | CN | OH | H | Me | CN | CH | |
| 4.143 | CN | Me | H | H | CN | CH | |
| 4.144 | CN | OMe | H | H | CN | CH | |
| 4.145 | OMe | H | H | H | CF₃ | N | |
| 4.146 | OMe | H | H | H | CF₃ | CH | ¹H NMR (CDCl₃/TMS): δ(ppm) 8.42 (s, 1H), 7.85 (s, 1H), 7.40 (d, 1H), 6.44 (d, 1H,), 6.29 (s, 1H), 3.99 (s, 3H), 3.81 (s, 3H). |
| 4.147 | OMe | H | H | H | CF₃ | CMe | |
| 4.148 | OMe | H | H | H | CF₃ | CCl | |
| 4.149 | OMe | H | Me | H | CF₃ | CH | |
| 4.150 | OMe | OH | H | Me | CF₃ | CH | |
| 4.151 | OMe | Me | H | H | CF₃ | CH | |
| 4.152 | OMe | OMe | H | H | CF₃ | CH | |
| 4.153 | OMe | H | H | H | Cl | N | |
| 4.154 | OMe | H | H | H | Cl | CH | |
| 4.155 | OMe | H | H | H | Cl | CMe | |
| 4.156 | OMe | H | H | H | Cl | CCl | |
| 4.157 | OMe | H | Me | H | Cl | CH | |
| 4.158 | OMe | OH | H | Me | Cl | CH | |
| 4.159 | OMe | Me | H | H | Cl | CH | |
| 4.160 | OMe | OMe | H | H | Cl | CH | |
| 4.161 | OMe | H | H | H | CN | N | |
| 4.162 | OMe | H | H | H | CN | CH | |
| 4.163 | OMe | H | H | H | CN | CMe | |
| 4.164 | OMe | H | H | H | CN | CCl | |
| 4.165 | OMe | H | Me | H | CN | CH | |
| 4.166 | OMe | OH | H | Me | CN | CH | |
| 4.167 | OMe | Me | H | H | CN | CH | |
| 4.168 | OMe | OMe | H | H | CN | CH | |
| 4.169 | OMe | H | H | H | OCF₂H | N | |
| 4.170 | OMe | H | H | H | OCF₂H | CH | |
| 4.171 | OMe | H | H | H | OCF₂H | CMe | |
| 4.172 | OMe | H | H | H | OCF₂H | CCI | |
| 4.173 | OMe | H | Me | H | OCF₂H | CH | |
| 4.174 | OMe | OH | H | Me | OCF₂H | CH | |
| 4.175 | OMe | Me | H | H | OCF₂H | CH | |
| 4.176 | OMe | OMe | H | H | OCF₂H | CH | |
| 4.177 | OMe | H | H | H | CN | N | |
| 4.178 | OMe | H | H | H | CN | CH | |
| 4.179 | OMe | H | H | H | CN | CMe | |
| 4.180 | OMe | H | H | H | CN | CCl | |
| 4.181 | OMe | H | Me | H | CN | CH | |
| 4.182 | OMe | OH | H | Me | CN | CH | |
| 4.183 | OMe | Me | H | H | CN | CH | |
| 4.184 | OMe | OMe | H | H | CN | CH | |
| 4.185 | OMe | H | H | H | CN | N | |
| 4.186 | OMe | H | H | H | CN | CH | |
| 4.187 | OMe | H | H | H | CN | CMe | |
| 4.188 | OMe | H | H | H | CN | CCl | |
| 4.189 | OMe | H | Me | H | CN | CH | |
| 4.190 | OMe | OH | H | Me | CN | CH | |
| 4.191 | OMe | Me | H | H | CN | CH | |
| 4.192 | OMe | OMe | H | H | CN | CH | |
| 4.193 | H | Cl | H | H | CF₃ | N | Fp. 113-115°C |
| 4.194 | Et | H | H | H | CF₃ | N | Fp. 106-108°C |
| 4.195 | Et | H | Me | H | CF₃ | N | Fp. 121-123°C |
| 4.196 | Et | H | H | Me | CF₃ | N | ¹H-NMR [CDCl₃]: 1.40 (t, 3H); 2.95 (9, 2H); 2.50 (s, 3H); 3.80 (s, 3H); 6.45 (s, 1H); 6.90 (s, 1H); 7.88 (s, 1H) |

**Tabelle 5: Erfindungsgemäße Verbindungen der allgemeinen Formel (I), worin die Substituenten und Symbole folgende Bedeutungen haben:**

| | | | | | | | |
|---|---|---|---|---|---|---|---|
| 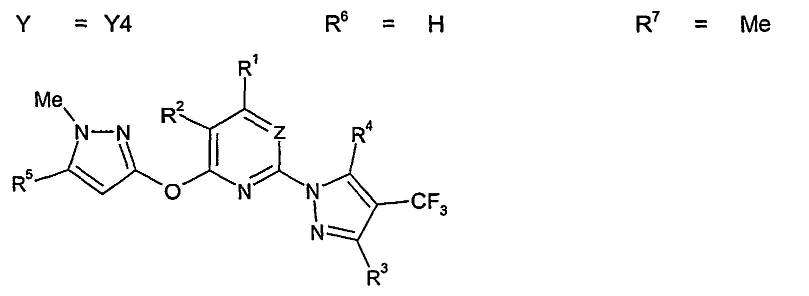 | | | | | | | |

| **Nr.** | **R**^{**1**} | **R**^{**2**} | **R**^{**3**} | **R**^{**4**} | **R**^{**5**} | **Z** | **Physikal. Daten** |
|---|---|---|---|---|---|---|---|
| 5.1 | H | H | H | H | CF₃ | N | |
| 5.2 | H | H | H | H | CF₃ | CH | |
| 5.3 | H | H | H | H | CF₃ | CMe | |
| 5.4 | H | H | H | H | CF₃ | CCl | |
| 5.5 | H | H | Me | H | CF₃ | CH | |
| 5.6 | H | OH | H | Me | CF₃ | CH | |
| 5.7 | H | Me | H | H | CF₃ | CH | |
| 5.8 | H | OMe | H | H | CF₃ | CH | |
| 5.9 | H | H | H | H | Cl | N | |
| 5.10 | H | H | H | H | Cl | CH | |
| 5.11 | H | H | H | H | Cl | CMe | |
| 5.12 | H | H | H | H | Cl | CCl | |
| 5.13 | H | H | Me | H | Cl | CH | |
| 5.14 | H | OH | H | Me | Cl | CH | |
| 5.15 | H | Me | H | H | Cl | CH | |
| 5.16 | H | OMe | H | H | Cl | CH | |
| 5.17 | H | H | H | H | CN | N | |
| 5.18 | H | H | H | H | CN | CH | |
| 5.19 | H | H | H | H | CN | CMe | |
| 5.20 | H | H | H | H | CN | CCl | |
| 5.21 | H | H | Me | H | CN | CH | |
| 5.22 | H | OH | H | Me | CN | CH | |
| 5.23 | H | Me | H | H | CN | CH | |
| 5.24 | H | OMe | H | H | CN | CH | |
| 5.25 | H | H | H | H | OCF₂H | N | |
| 5.26 | H | H | H | H | OCF₂H | CH | |
| 5.27 | H | H | H | H | OCF₂H | CMe | |
| 5.28 | H | H | H | H | OCF₂H | CCl | |
| 5.29 | H | H | Me | H | OCF₂H | CH | |
| 5.30 | H | OH | H | Me | OCF₂H | CH | |
| 5.31 | H | Me | H | H | OCF₂H | CH | |
| 5.32 | H | OMe | H | H | OCF₂H | CH | |
| 5.33 | H | H | H | H | CN | N | |
| 5.34 | H | H | H | H | CN | CH | |
| 5.35 | H | H | H | H | CN | CMe | |
| 5.36 | H | H | H | H | CN | CCl | |
| 5.37 | H | H | Me | H | CN | CH | |
| 5.38 | H | OH | H | Me | CN | CH | |
| 5.39 | H | Me | H | H | CN | CH | |
| 5.40 | H | OMe | H | H | CN | CH | |
| 5.41 | H | H | H | H | CN | N | |
| 5.42 | H | H | H | H | CN | CH | |
| 5.43 | H | H | H | H | CN | CMe | |
| 5.44 | H | H | H | H | CN | CCl | |
| 5.45 | H | H | Me | H | CN | CH | |
| 5.46 | H | OH | H | Me | CN | CH | |
| 5.47 | H | Me | H | H | CN | CH | |
| 5.48 | H | OMe | H | H | CN | CH | |
| 5.49 | Me | H | H | H | CF₃ | N | |
| 5.50 | Me | H | H | H | CF₃ | CH | |
| 5.51 | Me | H | H | H | CF₃ | CMe | |
| 5.52 | Me | H | H | H | CF₃ | CCl | |
| 5.53 | Me | H | Me | H | CF₃ | CH | |
| 5.54 | Me | OH | H | Me | CF₃ | CH | |
| 5.55 | Me | Me | H | H | CF₃ | CH | |
| 5.56 | Me | OMe | H | H | CF₃ | CH | |
| 5.57 | Me | H | H | H | Cl | N | |
| 5.58 | Me | H | H | H | Cl | CH | |
| 5.59 | Me | H | H | H | Cl | CMe | |
| 5.60 | Me | H | H | H | Cl | CCl | |
| 5.61 | Me | H | Me | H | Cl | CH | |
| 5.62 | Me | OH | H | Me | Cl | CH | |
| 5.63 | Me | Me | H | H | Cl | CH | |
| 5.64 | Me | OMe | H | H | Cl | CH | |
| 5.65 | Me | H | H | H | CN | N | |
| 5.66 | Me | H | H | H | CN | CH | |
| 5.67 | Me | H | H | H | CN | CMe | |
| 5.68 | Me | H | H | H | CN | CCl | |
| 5.69 | Me | H | Me | H | CN | CH | |
| 5.70 | Me | OH | H | Me | CN | CH | |
| 5.71 | Me | Me | H | H | CN | CH | |
| 5.72 | Me | OMe | H | H | CN | CH | |
| 5.73 | Me | H | H | H | OCF₂H | N | |
| 5.74 | Me | H | H | H | OCF₂H | CH | |
| 5.75 | Me | H | H | H | OCF₂H | CMe | |
| 5.76 | Me | H | H | H | OCF₂H | CCl | |
| 5.77 | Me | H | Me | H | OCF₂H | CH | |
| 5.78 | Me | OH | H | Me | OCF₂H | CH | |
| 5.79 | Me | Me | H | H | OCF₂H | CH | |
| 5.80 | Me | OMe | H | H | OCF₂H | CH | |
| 5.81 | Me | H | H | H | CN | N | |
| 5.82 | Me | H | H | H | CN | CH | |
| 5.83 | Me | H | H | H | CN | CMe | |
| 5.84 | Me | H | H | H | CN | CCl | |
| 5.85 | Me | H | Me | H | CN | CH | |
| 5.86 | Me | OH | H | Me | CN | CH | |
| 5.87 | Me | Me | H | H | CN | CH | |
| 5.88 | Me | OMe | H | H | CN | CH | |
| 5.89 | Me | H | H | H | CN | N | |
| 5.90 | Me | H | H | H | CN | CH | |
| 5.91 | Me | H | H | H | CN | CMe | |
| 5.92 | Me | H | H | H | CN | CCl | |
| 5.93 | Me | H | Me | H | CN | CH | |
| 5.94 | Me | OH | H | Me | CN | CH | |
| 5.95 | Me | Me | H | H | CN | CH | |
| 5.96 | Me | OMe | H | H | CN | CH | |
| 5.97 | CN | H | H | H | CF₃ | N | |
| 5.98 | CN | H | H | H | CF₃ | CH | |
| 5.99 | CN | H | H | H | CF₃ | CMe | |
| 5.100 | CN | H | H | H | CF₃ | CCl | |
| 5.101 | CN | H | Me | H | CF₃ | CH | |
| 5.102 | CN | OH | H | Me | CF₃ | CH | |
| 5.103 | CN | Me | H | H | CF₃ | CH | |
| 5.104 | CN | OMe | H | H | CF₃ | CH | |
| 5.105 | CN | H | H | H | Cl | N | |
| 5.106 | CN | H | H | H | Cl | CH | |
| 5.107 | CN | H | H | H | Cl | CMe | |
| 5.108 | CN | H | H | H | Cl | CCl | |
| 5.109 | CN | H | Me | H | Cl | CH | |
| 5.110 | CN | OH | H | Me | Cl | CH | |
| 5.111 | CN | Me | H | H | Cl | CH | |
| 5.112 | CN | OMe | H | H | Cl | CH | |
| 5.113 | CN | H | H | H | CN | N | |
| 5.114 | CN | H | H | H | CN | CH | |
| 5.115 | CN | H | H | H | CN | CMe | |
| 5.116 | CN | H | H | H | CN | CCl | |
| 5.117 | CN | H | Me | H | CN | CH | |
| 5.118 | CN | OH | H | Me | CN | CH | |
| 5.119 | CN | Me | H | H | CN | CH | |
| 5.120 | CN | OMe | H | H | CN | CH | |
| 5.121 | CN | H | H | H | OCF₂H | N | |
| 5.122 | CN | H | H | H | OCF₂H | CH | |
| 5.123 | CN | H | H | H | OCF₂H | CMe | |
| 5.124 | CN | H | H | H | OCF₂H | CCl | |
| 5.125 | CN | H | Me | H | OCF₂H | CH | |
| 5.126 | CN | OH | H | Me | OCF₂H | CH | |
| 5.127 | CN | Me | H | H | OCF₂H | CH | |
| 5.128 | CN | OMe | H | H | OCF₂H | CH | |
| 5.129 | CN | H | H | H | CN | N | |
| 5.130 | CN | H | H | H | CN | CH | |
| 5.131 | CN | H | H | H | CN | CMe | |
| 5.132 | CN | H | H | H | CN | CCl | |
| 5.133 | CN | H | Me | H | CN | CH | |
| 5.134 | CN | OH | H | Me | CN | CH | |
| 5.135 | CN | Me | H | H | CN | CH | |
| 5.136 | CN | OMe | H | H | CN | CH | |
| 5.137 | CN | H | H | H | CN | N | |
| 5.138 | CN | H | H | H | CN | CH | |
| 5.139 | CN | H | H | H | CN | CMe | |
| 5.140 | CN | H | H | H | CN | CCl | |
| 5.141 | CN | H | Me | H | CN | CH | |
| 5.142 | CN | OH | H | Me | CN | CH | |
| 5.143 | CN | Me | H | H | CN | CH | |
| 5.144 | CN | OMe | H | H | CN | CH | |
| 5.145 | OMe | H | H | H | CF₃ | N | |
| 5.146 | OMe | H | H | H | CF₃ | CH | |
| 5.147 | OMe | H | H | H | CF₃ | CMe | |
| 5.148 | OMe | H | H | H | CF₃ | CCl | |
| 5.149 | OMe | H | Me | H | CF₃ | CH | |
| 5.150 | OMe | OH | H | Me | CF₃ | CH | |
| 5.151 | OMe | Me | H | H | CF₃ | CH | |
| 5.152 | OMe | OMe | H | H | CF₃ | CH | |
| 5.153 | OMe | H | H | H | Cl | N | |
| 5.154 | OMe | H | H | H | Cl | CH | |
| 5.155 | OMe | H | H | H | Cl | CMe | |
| 5.156 | OMe | H | H | H | Cl | CCl | |
| 5.157 | OMe | H | Me | H | Cl | CH | |
| 5.158 | OMe | OH | H | Me | Cl | CH | |
| 5.159 | OMe | Me | H | H | Cl | CH | |
| 5.160 | OMe | OMe | H | H | Cl | CH | |
| 5.161 | OMe | H | H | H | CN | N | |
| 5.162 | OMe | H | H | H | CN | CH | |
| 5.163 | OMe | H | H | H | CN | CMe | |
| 5.164 | OMe | H | H | H | CN | CCl | |
| 5.165 | OMe | H | Me | H | CN | CH | |
| 5.166 | OMe | OH | H | Me | CN | CH | |
| 5.167 | OMe | Me | H | H | CN | CH | |
| 5.168 | OMe | OMe | H | H | CN | CH | |
| 5.169 | OMe | H | H | H | OCF₂H | N | |
| 5.170 | OMe | H | H | H | OCF₂H | CH | |
| 5.171 | OMe | H | H | H | OCF₂H | CMe | |
| 5.172 | OMe | H | H | H | OCF₂H | CCl | |
| 5.173 | OMe | H | Me | H | OCF₂H | CH | |
| 5.174 | OMe | OH | H | Me | OCF₂H | CH | |
| 5.175 | OMe | Me | H | H | OCF₂H | CH | |
| 5.176 | OMe | OMe | H | H | OCF₂H | CH | |
| 5.177 | OMe | H | H | H | CN | N | |
| 5.178 | OMe | H | H | H | CN | CH | |
| 5.179 | OMe | H | H | H | CN | CMe | |
| 5.180 | OMe | H | H | H | CN | CCl | |
| 5.181 | OMe | H | Me | H | CN | CH | |
| 5.182 | OMe | OH | H | Me | CN | CH | |
| 5.183 | OMe | Me | H | H | CN | CH | |
| 5.184 | OMe | OMe | H | H | CN | CH | |
| 5.185 | OMe | H | H | H | CN | N | |
| 5.186 | OMe | H | H | H | CN | CH | |
| 5.187 | OMe | H | H | H | CN | CMe | |
| 5.188 | OMe | H | H | H | CN | CCl | |
| 5.189 | OMe | H | Me | H | CN | CH | |
| 5.190 | OMe | OH | H | Me | CN | CH | |
| 5.191 | OMe | Me | H | H | CN | CH | |
| 5.192 | OMe | OMe | H | H | CN | CH | |

**Tabelle 6: Erfindungsgemäße Verbindungen der allgemeinen Formel (I), worin die Substituenten und Symbole folgende Bedeutungen haben:**

| | | | | | | | |
|---|---|---|---|---|---|---|---|
| 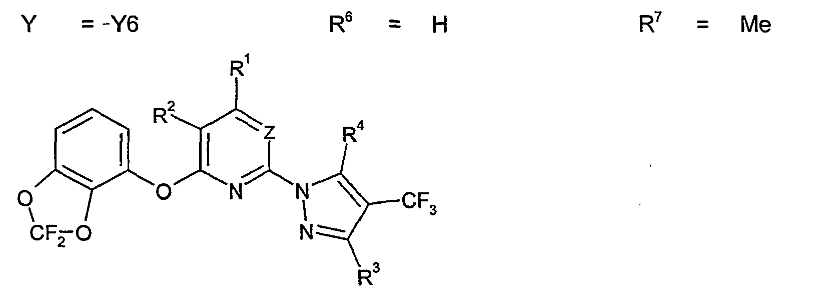 | | | | | | | |

| **Nr.** | **R**^{**1**} | **R**^{**2**} | **R**^{**3**} | **R**^{**4**} | **R**^{**5**} | **Z** | **Physikal. Daten** |
|---|---|---|---|---|---|---|---|
| 6.1 | H | H | H | H | CF₃ | N | |
| 6.2 | H | H | H | H | CF₃ | CH | |
| 6.3 | H | H | H | H | CF₃ | CMe | |
| 6.4 | H | H | H | H | CF₃ | CCl | |
| 6.5 | H | H | Me | H | CF₃ | CH | |
| 6.6 | H | OH | H | Me | CF₃ | CH | |
| 6.7 | H | Me | H | H | CF₃ | CH | |
| 6.8 | H | OMe | H | H | CF₃ | CH | |
| 6.9 | H | H | H | H | Cl | N | |
| 6.10 | H | H | H | H | Cl | CH | |
| 6.11 | H | H | H | H | Cl | CMe | |
| 6.12 | H | H | H | H | Cl | CCl | |
| 6.13 | H | H | Me | H | Cl | CH | |
| 6.14 | H | OH | H | Me | Cl | CH | |
| 6.15 | H | Me | H | H | Cl | CH | |
| 6.16 | H | OMe | H | H | Cl | CH | |
| 6.17 | H | H | H | H | CN | N | |
| 6.18 | H | H | H | H | CN | CH | |
| 6.19 | H | H | H | H | CN | CMe | |
| 6.20 | H | H | H | H | CN | CCl | |
| 6.21 | H | H | Me | H | CN | CH | |
| 6.22 | H | OH | H | Me | CN | CH | |
| 6.23 | H | Me | H | H | CN | CH | |
| 6.24 | H | OMe | H | H | CN | CH | |
| 6.25 | H | H | H | H | OCF₂H | N | |
| 6.26 | H | H | H | H | OCF₂H | CH | |
| 6.27 | H | H | H | H | OCF₂H | CMe | |
| 6.28 | H | H | H | H | OCF₂H | CCl | |
| 6.29 | H | H | Me | H | OCF₂H | CH | |
| 6.30 | H | OH | H | Me | OCF₂H | CH | |
| 6.31 | H | Me | H | H | OCF₂H | CH | |
| 6.32 | H | OMe | H | H | OCF₂H | CH | |
| 6.33 | H | H | H | H | CN | N | |
| 6.34 | H | H | H | H | CN | CH | |
| 6.35 | H | H | H | H | CN | CMe | |
| 6.36 | H | H | H | H | CN | CCl | |
| 6.37 | H | H | Me | H | CN | CH | |
| 6.38 | H | OH | H | Me | CN | CH | |
| 6.39 | H | Me | H | H | CN | CH | |
| 6.40 | H | OMe | H | H | CN | CH | |
| 6.41 | H | H | H | H | CN | N | |
| 6.42 | H | H | H | H | CN | CH | |
| 6.43 | H | H | H | H | CN | CMe | |
| 6.44 | H | H | H | H | CN | CCl | |
| 6.45 | H | H | Me | H | CN | CH | |
| 6.46 | H | OH | H | Me | CN | CH | |
| 6.47 | H | Me | H | H | CN | CH | |
| 6.48 | H | OMe | H | H | CN | CH | |
| 6.49 | Me | H | H | H | CF₃ | N | |
| 6.50 | Me | H | H | H | CF₃ | CH | |
| 6.51 | Me | H | H | H | CF₃ | CMe | |
| 6.52 | Me | H | H | H | CF₃ | CCl | |
| 6.53 | Me | H | Me | H | CF₃ | CH | |
| 6.54 | Me | OH | H | Me | CF₃ | CH | |
| 6.55 | Me | Me | H | H | CF₃ | CH | |
| 6.56 | Me | OMe | H | H | CF₃ | CH | |
| 6.57 | Me | H | H | H | Cl | N | |
| 6.58 | Me | H | H | H | Cl | CH | |
| 6.59 | Me | H | H | H | Cl | CMe | |
| 6.60 | Me | H | H | H | Cl | CCl | |
| 6.61 | Me | H | Me | H | Cl | CH | |
| 6.62 | Me | OH | H | Me | Cl | CH | |
| 6.63 | Me | Me | H | H | Cl | CH | |
| 6.64 | Me | OMe | H | H | Cl | CH | |
| 6.65 | Me | H | H | H | CN | N | |
| 6.66 | Me | H | H | H | CN | CH | |
| 6.67 | Me | H | H | H | CN | CMe | |
| 6.68 | Me | H | H | H | CN | CCl | |
| 6.69 | Me | H | Me | H | CN | CH | |
| 6.70 | Me | OH | H | Me | CN | CH | |
| 6.71 | Me | Me | H | H | CN | CH | |
| 6.72 | Me | OMe | H | H | CN | CH | |
| 6.73 | Me | H | H | H | OCF₂H | N | |
| 6.74 | Me | H | H | H | OCF₂H | CH | |
| 6.75 | Me | H | H | H | OCF₂H | CMe | |
| 6.76 | Me | H | H | H | OCF₂H | CCl | |
| 6.77 | Me | H | Me | H | OCF₂H | CH | |
| 6.78 | Me | OH | H | Me | OCF₂H | CH | |
| 6.79 | Me | Me | H | H | OCF₂H | CH | |
| 6.80 | Me | OMe | H | H | OCF₂H | CH | |
| 6.81 | Me | H | H | H | CN | N | |
| 6.82 | Me | H | H | H | CN | CH | |
| 6.83 | Me | H | H | H | CN | CMe | |
| 6.84 | Me | H | H | H | CN | CCl | |
| 6.85 | Me | H | Me | H | CN | CH | |
| 6.86 | Me | OH | H | Me | CN | CH | |
| 6.87 | Me | Me | H | H | CN | CH | |
| 6.88 | Me | OMe | H | H | CN | CH | |
| 6.89 | Me | H | H | H | CN | N | |
| 6.90 | Me | H | H | H | CN | CH | |
| 6.91 | Me | H | H | H | CN | CMe | |
| 6.92 | Me | H | H | H | CN | CCl | |
| 6.93 | Me | H | Me | H | CN | CH | |
| 6.94 | Me | OH | H | Me | CN | CH | |
| 6.95 | Me | Me | H | H | CN | CH | |
| 6.96 | Me | OMe | H | H | CN | CH | |
| 6.97 | CN | H | H | H | CF₃ | N | |
| 6.98 | CN | H | H | H | CF₃ | CH | |
| 6.99 | CN | H | H | H | CF₃ | CMe | |
| 6.100 | CN | H | H | H | CF₃ | CCl | |
| 6.101 | CN | H | Me | H | CF₃ | CH | |
| 6.102 | CN | OH | H | Me | CF₃ | CH | |
| 6.103 | CN | Me | H | H | CF₃ | CH | |
| 6.104 | CN | OMe | H | H | CF₃ | CH | |
| 6.105 | CN | H | H | H | Cl | N | |
| 6.106 | CN | H | H | H | Cl | CH | |
| 6.107 | CN | H | H | H | Cl | CMe | |
| 6.108 | CN | H | H | H | Cl | CCl | |
| 6.109 | CN | H | Me | H | Cl | CH | |
| 6.110 | CN | OH | H | Me | Cl | CH | |
| 6.111 | CN | Me | H | H | Cl | CH | |
| 6.112 | CN | OMe | H | H | Cl | CH | |
| 6.113 | CN | H | H | H | CN | N | |
| 6.114 | CN | H | H | H | CN | CH | |
| 6.115 | CN | H | H | H | CN | CMe | |
| 6.116 | CN | H | H | H | CN | CCl | |
| 6.117 | CN | H | Me | H | CN | CH | |
| 6.118 | CN | OH | H | Me | CN | CH | |
| 6.119 | CN | Me | H | H | CN | CH | |
| 6.120 | CN | OMe | H | H | CN | CH | |
| 6.121 | CN | H | H | H | OCF₂H | N | |
| 6.122 | CN | H | H | H | OCF₂H | CH | |
| 6.123 | CN | H | H | H | OCF₂H | CMe | |
| 6.124 | CN | H | H | H | OCF₂H | CCl | |
| 6.125 | CN | H | Me | H | OCF₂H | CH | |
| 6.126 | CN | OH | H | Me | OCF₂H | CH | |
| 6.127 | CN | Me | H | H | OCF₂H | CH | |
| 6.128 | CN | OMe | H | H | OCF₂H | CH | |
| 6.129 | CN | H | H | H | CN | N | |
| 6.130 | CN | H | H | H | CN | CH | |
| 6.131 | CN | H | H | H | CN | CMe | |
| 6.132 | CN | H | H | H | CN | CCl | |
| 6.133 | CN | H | Me | H | CN | CH | |
| 6.134 | CN | OH | H | Me | CN | CH | |
| 6.135 | CN | Me | H | H | CN | CH | |
| 6.136 | CN | OMe | H | H | CN | CH | |
| 6.137 | CN | H | H | H | CN | N | |
| 6.138 | CN | H | H | H | CN | CH | |
| 6.139 | CN | H | H | H | CN | CMe | |
| 6.140 | CN | H | H | H | CN | CCl | |
| 6.141 | CN | H | Me | H | CN | CH | |
| 6.142 | CN | OH | H | Me | CN | CH | |
| 6.143 | CN | Me | H | H | CN | CH | |
| 6.144 | CN | OMe | H | H | CN | CH | |
| 6.145 | OMe | H | H | H | CF₃ | N | |
| 6.146 | OMe | H | H | H | CF₃ | CH | |
| 6.147 | OMe | H | H | H | CF₃ | CMe | |
| 6.148 | OMe | H | H | H | CF₃ | CCl | |
| 6.149 | OMe | H | Me | H | CF₃ | CH | |
| 6.150 | OMe | OH | H | Me | CF₃ | CH | |
| 6.151 | OMe | Me | H | H | CF₃ | CH | |
| 6.152 | OMe | OMe | H | H | CF₃ | CH | |
| 6.153 | OMe | H | H | H | Cl | N | |
| 6.154 | OMe | H | H | H | Cl | CH | |
| 6.155 | OMe | H | H | H | Cl | CMe | |
| 6.156 | OMe | H | H | H | Cl | CCl | |
| 6.157 | OMe | H | Me | H | Cl | CH | |
| 6.158 | OMe | OH | H | Me | Cl | CH | |
| 6.159 | OMe | Me | H | H | Cl | CH | |
| 6.160 | OMe | OMe | H | H | Cl | CH | |
| 6.161 | OMe | H | H | H | CN | N | |
| 6.162 | OMe | H | H | H | CN | CH | |
| 6.163 | OMe | H | H | H | CN | CMe | |
| 6.164 | OMe | H | H | H | CN | CCl | |
| 6.165 | OMe | H | Me | H | CN | CH | |
| 6.166 | OMe | OH | H | Me | CN | CH | |
| 6.167 | OMe | Me | H | H | CN | CH | |
| 6.168 | OMe | OMe | H | H | CN | CH | |
| 6.169 | OMe | H | H | H | OCF₂H | N | |
| 6.170 | OMe | H | H | H | OCF₂H | CH | |
| 6.171 | OMe | H | H | H | OCF₂H | CMe | |
| 6.172 | OMe | H | H | H | OCF₂H | CCl | |
| 6.173 | OMe | H | Me | H | OCF₂H | CH | |
| 6.174 | OMe | OH | H | Me | OCF₂H | CH | |
| 6.175 | OMe | Me | H | H | OCF₂H | CH | |
| 6.176 | OMe | OMe | H | H | OCF₂H | CH | |
| 6.177 | OMe | H | H | H | CN | N | |
| 6.178 | OMe | H | H | H | CN | CH | |
| 6.179 | OMe | H | H | H | CN | CMe | |
| 6.180 | OMe | H | H | H | CN | CCl | |
| 6.181 | OMe | H | Me | H | CN | CH | |
| 6.182 | OMe | OH | H | Me | CN | CH | |
| 6.183 | OMe | Me | H | H | CN | CH | |
| 6.184 | OMe | OMe | H | H | CN | CH | |
| 6.185 | OMe | H | H | H | CN | N | |
| 6.186 | OMe | H | H | H | CN | CH | |
| 6.187 | OMe | H | H | H | CN | CMe | |
| 6.188 | OMe | H | H | H | CN | CCl | |
| 6.189 | OMe | H | Me | H | CN | CH | |
| 6.190 | OMe | OH | H | Me | CN | CH | |
| 6.191 | OMe | Me | H | H | CN | CH | |
| 6.192 | OMe | OMe | H | H | CN | CH | |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der allgemeinen Formel(I),
10 " ligninsulfonsaures Calcium,
5 " Natriumlaurylsulfat,
3 " Polyvinylalkohol und
7 " Kaolin
mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man 25 Gew.-Teile einer Verbindung der allgemeinen Formel (I),
5 " 2,2'-dinaphthylmethan-6,6'-disulfonsaures Natrium,
2 " oleoylmethyltaurinsaures Natrium,
1 " Polyvinylalkohol,
17 " Calciumcarbonat und
50 " Wasser
auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen sowie Kulturpflanzenverträglichkeit im Vorauflauf

Samen von mono- und dikotylen Unkrautpflanzen sowie von Kulturpflanzen werden in Papptöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 I/ha in unterschiedlichen Dosierungen auf die Oberfläche der Abdeckerde appliziert. Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Dabei zeigen beispielsweise die erfindungsgemäßen Verbindungen der Beispiele Nr. 1.7 und 4.1 bei einer Dosierung von 320 g Aktivsubstanz pro Hektar eine 100 %ige Wirkung gegen Digitaria sanguinalis, Setaria viridis und Amaranthus retroflexus. Bei gleicher Dosierung verursachen diese erfindungsgemäßen Verbindungen keine Schädigung an den Kulturpflanzen Oryza sativa (Reis) und Glycine max (Sojabohne). Die erfindungsgemäße Verbindung des Beispiels Nr. 1.7 zeigt bei einer Dosierung von 20 g Aktivsubstanz pro Hektar eine mindestens 90 %ige Wirkung gegen Alopecurus myosuroides, Setaria viridis, Amaranthus retroflexus und Veronica persica. Bei gleicher Dosierung verursacht diese erfindungsgemäße Verbindung keine Schädigung an den Kulturpflanzen Oryza sativa (Reis), Zea mays (Mais) und Glycine max (Sojabohne). Bei einer Dosierung von 320 g Aktivsubstanz pro Hektar zeigt die erfindungsgemäße Verbindung des Beispiels Nr. 4.146 eine 100 %ige Wirkung gegen Amaranthus retroflexus, Setaria viridis und Stellaria media.

### 2. Herbizide Wirkung gegen Schadpflanzen sowie Kulturpflanzenverträglichkeit im Nachauflauf

Samen von mono- und dikotylen Schadpflanzen sowie von Kulturpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha in unterschiedlichen Dosierungen auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen bonitiert. Dabei zeigen beispielsweise bei einer Dosierung von 80 g Aktivsubstanz pro Hektar die erfindungsgemäßen Verbindungen der Beispiele Nr. 4.1 und 4.49 eine mindestens 90 %ige Wirkung gegen Setaria viridis, Digitaria sanguinalis, Matricaria inodora, Amaranthus retroflexus, Pharbitis purpureum, Chenopodium album, Veronica persica und Abutilon theophrasti.

## Patentansprüche

1. Verbindungen der Formel (I), deren N-Oxide und deren Salze, 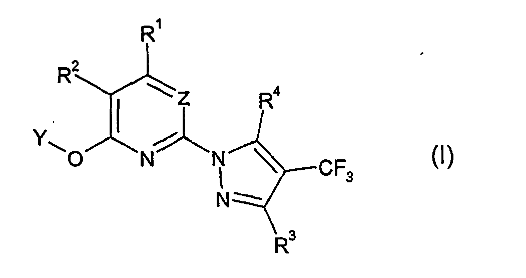 worin die Reste und Indizes folgende Bedeutungen haben:
Z bedeutet N oder CR⁸;
Y bedeutet einen Rest aus der Gruppe Y1 bisY7: 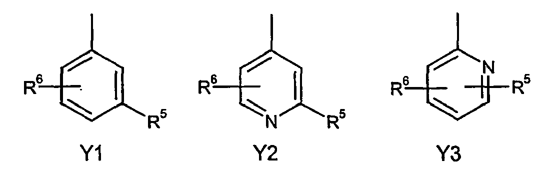 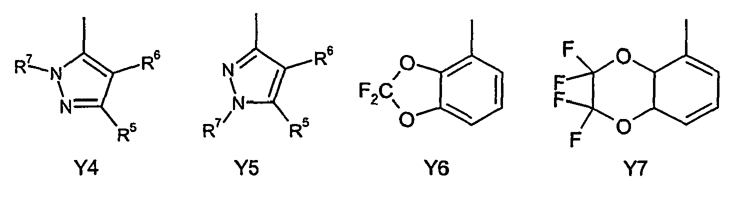
R¹ und R² bedeuten unabhängig voneinander Wasserstoff, Halogen, Cyano, Isocyano, OH, COOR¹⁰, COR¹⁰, CH₂OH, CH₂SH, CH₂NH₂, NO₂, CSNH₂, CONH₂, (C₋₁-C₄)-Alkyl, Halogen-(C₁-C₄)-alkyl, (C₃-C₆)-Cycloalkyl, (C₁-C₄)-Alkoxy, Halogen-(C₁₋C₄)-alkoxy, (C₁-C₂)-Alkoxy-(C₁-C₂)-alkyl, (C₂-C₄)-Alkenyl, (C₂-C₄)-Alkinyl, (C₃-C₄)-Alkenyloxy, (C₃-C₄)-Alkinyloxy, (C₁-C₂)-Alkylthio-(C₁-C₂)-alkyl, S(O)ₙR⁹, (C₁-C₂)-Alkylsulfonyl-(C₁-C₂)-alkyl, Amino, (C₁-C₄)-Alkylamino, (C₁-C₃)-Alkylcarbonylamino, (C₁-C₄)-Alkylsulfonylamino oder Di-(C₁-C₄)-Alkylamino;
R³ und R⁴ bedeuten unabhängig voneinander Wasserstoff, Halogen, Cyano, (C₁₋C₄)-Alkyl, Halogen-(C₁-C₄)-alkyl, (C₁-C₄)-Alkoxy oder Halogen-(C₁-C₄)-alkoxy;
R⁵ bedeutet Halogen, Cyano, (C₁-C₄)-Alkyl, Halogen-(C₁-C₄)-alkyl, (C₁-C₄)-Alkoxy, Halogen-(C₁-C₄)-alkoxy, Halogen-(C₁-C₄)-alkylthio, (C₃-C₅)-Cycloalkyl, Halogen-(C₃-C₅)-Cycloalkyl, SF₅, S(O)ₙR⁹, (C₂-C₄)-Alkenyl oder (C₂-C₄)-Alkinyl;
R⁶ bedeutet Wasserstoff, Halogen, Cyano, (C₁-C₄)-Alkyl, Halogen-(C₁-C₄)-alkyl, (C₁-C₄)-Alkoxy, Halogen-(C₁-C₄)-alkoxy oder S(O)ₙR⁹;
R⁷ bedeutet (C₁-C₄)-Alkyl;
R⁸ bedeutet Wasserstoff, Halogen, Cyano, NO₂, (C₁-C₄)-Alkyl, (C₁-C₄)-Alkoxy, Hydroxy, Amino, (C₁-C₄)-Alkylamino, (C₁-C₃)-Alkylcarbonylamino, (C₁-C₄)-Alkylsulfonylamino, Di-(C₁-C₄)-Alkylamino oder S(O)ₙR⁹;
R⁹ bedeutet Wasserstoff, (C₁-C₄)-Alkyl oder Halogen-(C₁-C₄)-alkyl;
R¹⁰ bedeutet Wasserstoff oder (C₁-C₄)-Alkyl;
n bedeutet 0, 1 oder 2.

2. Verbindungen nach Anspruch 1, worin Y einen Rest aus der Gruppe Y1 bis Y6 bedeutet.

3. Verbindungen nach Anspruch 1 oder 2, worin R¹ und R² unabhängig voneinander Wasserstoff, Halogen, Cyano, OH, CHO, Vinyl, (C₁-C₄)-Alkyl, Halogen-(C₁-C₄)-Alkyl, Vinyl oder (C₁-C₄)-Alkoxy bedeuten.

4. Verbindungen nach einem der Ansprüche 1 bis 3, worin R³ und R⁴ unabhängig voneinander Wasserstoff, Halogen, Methyl oder Methoxy bedeuten.

5. Verbindungen nach einem der Ansprüche 1 bis 4, worin
R¹ Wasserstoff, Halogen, Cyano, CHO, Methoxy, Methyl oder Ethyl, und
R² Wasserstoff, OH, Methyl, Ethyl, Methoxy oder Ethoxy bedeuten.

6. Verbindungen nach einem der Ansprüche 1 bis 5, worin R³ und R⁴ jeweils Wasserstoff oder Methyl bedeuten.

7. Verbindungen nach einem der Ansprüche 1 bis 6, worin R⁸ für Wasserstoff, Halogen oder (C₁-C₄)-Alkyl steht..

8. Verbindungen nach einem der Ansprüche 1 bis 7, worin R⁵ für Halogen, Cyano, Halogen-(C₁-C₄)-alkyl, Halogen-(C₁-C₄)-alkoxy oder Halogen-(C₁-C₄)-alkylthio steht.

9. Verbindungen nach einem der Ansprüche 1 bis 8, worin R⁶ Wasserstoff bedeutet.

10. Herbizide Mittel, **gekennzeichnet durch** einen herbizid wirksamen Gehalt an mindestens einer Verbindung der allgemeinen Formel (I) gemäß einem der Ansprüche 1 bis 9.

11. Herbizide Mittel nach Anspruch 10 in Mischung mit Formulierungshilfsmitteln.

12. Verfahren zur Bekämpfung unerwünschter Pflanzen, **dadurch gekennzeichnet, daß** man eine wirksame Menge mindestens einer Verbindung der allgemeinen Formel (I) nach einem der Ansprüche 1 bis 9 oder eines herbiziden Mittels nach Anspruch 10 oder 11 auf die Pflanzen oder auf den Ort des unerwünschten Pflanzenwachstums appliziert.

13. Verwendung von Verbindungen der allgemeinen Formel (I) nach einem der Ansprüche 1 bis 9 oder von herbiziden Mitteln nach Anspruch 10 oder 11 zur Bekämpfung unerwünschter Pflanzen.

14. Verwendung nach Anspruch 13, **dadurch gekennzeichnet, daß** die Verbindungen der allgemeinen Formel (I) zur Bekämpfung unerwünschter Pflanzen in Kulturen von Nutzpflanzen eingesetzt werden.

15. Verwendung nach Anspruch 14, **dadurch gekennzeichnet, daß** die Nutzpflanzen transgene Nutzpflanzen sind.

## Claims

1. A compound of the formula (I) or N-oxide or salt thereof, 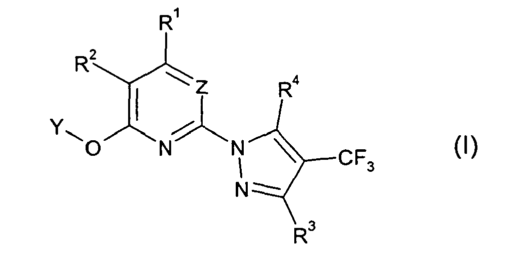 in which the radicals and indices have the following definitions:
Z is N or CR⁸;
Y is a radical from the group Y1 to Y7: 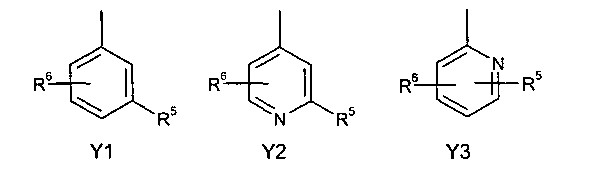 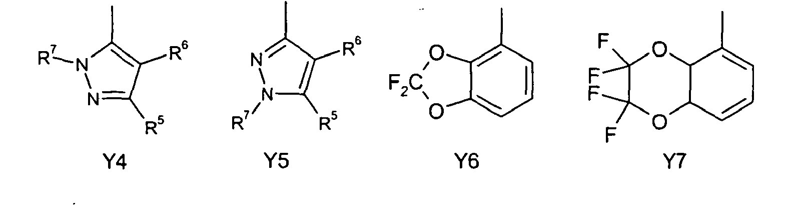
R¹ and R² independently are each hydrogen, halogen, cyano, isocyano, OH, COOR¹⁰, COR¹⁰, CH₂OH, CH₂SH, CH₂NH₂, NO₂, CSNH₂, CONH₂, (C₁-C₄)-alkyl, halo-(C₁-C₄)-alkyl, (C₃-C₆)-cycloalkyl, (C₁-C₄)-alkoxy, halo-(C₁-C₄)-alkoxy, (C₁₋C₂)-alkoxy-(C₁-C₂)-alkyl, (C₂-C₄)-alkenyl, (C₂-C₄)-alkynyl, (C₃-C₄)-alkenyloxy, (C₃-C₄)-alkynyloxy, (C₁-C₂)-alkylthio-(C₁-C₂)-alkyl, S(O)ₙR⁹, (C₁-C₂)-alkylsulfonyl-(C₁-C₂)-alkyl, amino, (C₁-C₄)-alkylamino, (C₁-C₃)-alkylcarbonylamino, (C₁-C₄)-alkylsulfonylamino or di-(C₁-C₄)-alkylamino;
R³ and R⁴ independently are each hydrogen, halogen, cyano, (C₁-C₄)-alkyl, halo-(C₁-C₄)-alkyl, (C₁-C₄)-alkoxy or halo-(C₁-C₄)-alkoxy;
R⁵ is halogen, cyano, (C₁-C₄)-alkyl, halo-(C₁-C₄)-alkyl, (C₁-C₄)-alkoxy, halo-(C₁₋C₄)-alkoxy, halo-(C₁-C₄)-alkylthio, (C₃-C₅)-cycloalkyl, halo-(C₃-C₅)-cycloalkyl, SF₅, S(O)ₙR⁹, (C₂-C₄)-alkenyl or (C₂-C₄)-alkynyl;
R⁶ is hydrogen, halogen, cyano, (C₁-C₄)-alkyl, halo-(C₁-C₄)-alkyl, (C₁-C₄)-alkoxy, halo-(C₁-C₄)-alkoxy or S(O)ₙR⁹;
R⁷ is (C₁-C₄)-alkyl;
R⁸ is hydrogen, halogen, cyano, NO₂, (C₁-C₄)-alkyl, (C₁-C₄)-alkoxy, hydroxy, amino, (C₁-C₄)-alkylamino, (C₁-C₃)-alkylcarbonylamino, (C₁-C₄)-alkylsulfonylamino, di-(C₁-C₄)-alkylamino or S(O)ₙR⁹;
R⁹ is hydrogen, (C₁-C₄)-alkyl or halo-(C₁-C₄)-alkyl;
R¹⁰ is hydrogen or (C₁-C₄)-alkyl;
n is 0, 1 or 2.

2. A compound as claimed in claim 1, wherein Y is a radical from the group Y1 to Y6.

3. A compound as claimed in claim 1 or 2, wherein R¹ and R² independently are each hydrogen, halogen, cyano, OH, CHO, vinyl, (C₁-C₄)-alkyl, halo-(C₁-C₄)-alkyl, vinyl or (C₁-C₄)-alkoxy.

4. A compound as claimed in any of claims 1 to 3, wherein R³ and R⁴ independently are each hydrogen, halogen, methyl or methoxy.

5. A compound as claimed in any of claims 1 to 4, wherein
R¹ is hydrogen, halogen, cyano, CHO, methoxy, methyl or ethyl, and
R² is hydrogen, OH, methyl, ethyl, methoxy or ethoxy.

6. A compound as claimed in any of claims 1 to 5, wherein R³ and R⁴ are each hydrogen or methyl.

7. A compound as claimed in any of claims 1 to 6, wherein R⁸ is hydrogen, halogen or (C₁-C₄)-alkyl.

8. A compound as claimed in any of claims 1 to 7, wherein R⁵ is halogen, cyano, halo-(C₁-C₄)-alkyl, halo-(C₁-C₄)-alkoxy or halo-(C₁-C₄)-alkylthio.

9. A compound as claimed in any of claims 1 to 8, wherein R⁶ is hydrogen.

10. A herbicidal composition comprising a herbicidally effective amount of at least one compound of the formula (I) as claimed in any of claims 1 to 9.

11. A herbicidal composition as claimed in claim 10 in a mixture with formulation auxiliaries.

12. A method of controlling unwanted plants, which comprises applying an effective amount of at least one compound of the formula (I) as claimed in any of claims 1 to 9 or of a herbicidal composition as claimed in claim 10 or 11 to the plants or to the site of the unwanted plant growth.

13. The use of a compound of the formula (I) as claimed in any of claims 1 to 9 or of a herbicidal composition as claimed in claim 10 or 11 to control unwanted plants.

14. The use as claimed in claim 13, wherein the compound of the formula (I) is used to control unwanted plants in crops of useful plants.

15. The use as claimed in claim 14, wherein the useful plants are transgenic.

## Revendications

1. Composés de formule (I), leurs N-oxydes et leurs sels 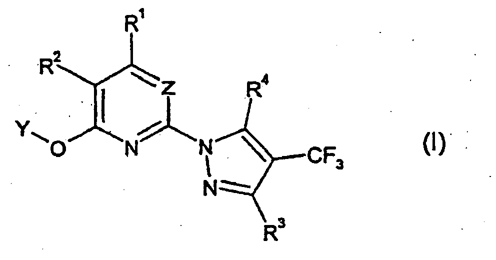 où les restes et les indices possèdent les significations suivantes :
Z représente un atome de N ou un groupe CR⁸;
Y représente un reste du groupe Y1 à Y7 : 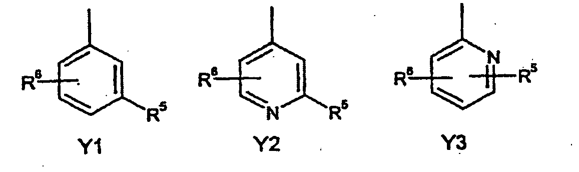 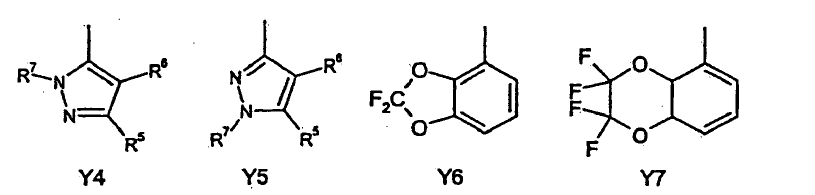
R¹ et R², indépendamment l'un de l'autre, représentent un atome d'hydrogène, un atome d'halogène, un groupe cyano, isocyano, OH, COOR¹⁰, COR¹⁰, CH₂OH, CH₂SH, CH₂NH₂, NO₂, CSNH₂, CONH₂, alkyle en C₁-C₄, halogénoalkyle en C₁-C₄, cycloalkyle en C₃-C₆, alkoxy en C₁-C₄, halogénoalkoxy en C₁-C₄, (alkoxy en C₁-C₂)-(alkyle en C₁-C₂), alcényle en C₂-C₄, alcynyle en C₂-C₄, (alcényl en C₃-C₄)oxy, (alcynyl en C₃-C₄)oxy, (alkyl en C₁-C₂)thio-(alkyl en C₁-C₂), S(O)ₙR⁹, (alkyl en C₁-C₂)-sulfonyl-(alkyle en C₁-C₂), amino, (alkyl en C₁-C₄)-amino, (alkyl en C₁-C₃)carbonylamino, (alkyl en C₁-C₄) sulfonylamino ou di (alkyl en C₁-C₄)-amino ;
R³ et R⁴, indépendamment l'un de l'autre, représentent un atome d'hydrogène, un atome d'halogène, un groupe cyano, alkyle en C₁-C₄, halogénoalkyle en C₁-C₄, alkoxy en C₁-C₄ ou halogénoalkoxy en C₁-C₄ ;
R⁵ représente un atome d'halogène, un groupe cyano, alkyle en C₁-C₄, halogénoalkyle en C₁-C₄, alkoxy en C₁-C₄, halogénoalkoxy en C₁-C₄, halogéno(alkyl en C₁-C₄)thio, cycloalkyle en C₃-C₅, halogénocycloalkyle en C₃-C₅, SF₅, S(O)ₙR⁹, alcényle en C₂-C₄ ou alcynyle en C₂-C₄ ;
R⁶ représente un atome d'hydrogène, un atome d'halogène, un groupe cyano, alkyle en C₁-C₄, halogénoalkyle en C₁-C₄, alkoxy en C₁-C₄, halogénoalkoxy en C₁-C₄, ou S(O)ₙR⁹;
R⁷ représente un groupe alkyle en C₁-C₄ ;
R⁸ représente un atome d'hydrogène, un atome d'halogène, un groupe cyano, NO₂, alkyle en C₁-C₄, alkoxy en C₁-C₄, hydroxy, amino, (alkyl en C₁-C₄)amino, (alkyl en C₁-C₃)carbonylamino, (alkyl en C₁-C₉)sulfonylamino, di(alkyl en C₁-C₄)-amino ou S(O)ₙR⁹ ;
R⁹ représente un atome d'hydrogène, un groupe alkyle en C₁-C₄ ou halogénoalkyle en C₁-C₄ ;
R¹⁰ représente un atome d'hydrogène ou un groupe alkyle en C₁-C₄ ;
n vaut 0, 1 oder 2.

2. Composés selon la revendication 1, dans lesquels Y représente un reste pris dans le groupe Y1 à Y6.

3. Composés selon la revendication 1 ou 2, dans lesquels R¹ et R², indépendamment l'un de l'autre, représentent un atome d'hydrogène, un atome d'halogène, un groupe cyano, OH, CHO, vinyle, alkyle en C₁-C₄, halogénoalkyle en C₁-C₄, vinyle ou alkoxy en C₁-C₄.

4. Composés selon la revendication 1 à 3, dans lesquels R³ et R⁴, indépendamment l'un de l'autre, représentent un atome d'hydrogène, un atome d'halogène, un groupe méthyle ou méthoxy.

5. Composés selon l'une des revendications 1 à 4, dans lesquels
R¹ représente un atome d'hydrogène, un atome d'halogène, un groupe cyano, CHO, méthoxy, méthyle ou éthyle, et
R² représente un atome d'hydrogène, un groupe OH méthyle, éthyle, méthoxy ou éthoxy.

6. Composés selon l'une des revendications 1 à 5, dans lesquels R³ et R⁴ représentent chacun un atome d'hydrogène ou un groupe méthyle.

7. Composés selon l'une des revendications 1 à 6, dans lesquels R⁸ représente un atome d'hydrogène, un atome d'halogène ou un groupe alkyle en C₁-C₄.

8. Composés selon l'une des revendications 1 à 7, dans lesquels R⁵ représente un atome d'halogène, un groupe cyano, halogénoalkyle en C₁-C₄, halogénoalkoxy en C₁-C₄ ou halogéno(alkyl en C₁-C₄)thio.

9. Composés selon l'une des revendications 1 à 8, dans lesquels R⁶ représente un atome d'hydrogène.

10. Agents herbicides **caractérisés par** une teneur à efficacité herbicide en au moins un composé de formule générale (I) selon l'une des revendications 1 à 9.

11. Agents herbicides selon la revendication 10 en mélange avec des matières auxiliaires formulantes.

12. Procédé pour la lutte contre les plantes indésirables, **caractérisé en ce qu'**on applique une quantité efficace d'au moins un composé de formule générale (I) selon l'une des revendications 1 à 9 ou d'un agent herbicide selon la revendication 10 ou 11, aux plantes ou à l'endroit de la croissance des plantes indésirable.

13. Utilisation de composés de formule générale (I) selon l'une des revendications 1 à 9 ou d'agents herbicides selon la revendication 10 ou 11 dans la lutte contre les plantes indésirable.

14. Utilisation selon la revendication 13, **caractérisée en ce qu'**on utilise les composés de formule générale (I) dans la lutte contre les plantes indésirables dans les cultures de plantes utiles.

15. Utilisation selon la revendication 14, **caractérisée en ce que** les plantes utiles sont les plantes utiles transgéniques.
